(19) Europäisches Patentamt
European Patent Office
Office européen des brevets

(11) **EP 2 384 124 B1**

(12) **EUROPEAN PATENT SPECIFICATION**

(45) Date of publication and mention
of the grant of the patent:
**16.09.2015 Bulletin 2015/38**

(21) Application number: **09796925.7**

(22) Date of filing: **30.12.2009**

(51) Int Cl.:
*A23J 3/16* (2006.01)     *A23J 3/34* (2006.01)
*A23L 1/305* (2006.01)     *A23C 20/00* (2006.01)
*A23L 2/39* (2006.01)     *A23L 2/66* (2006.01)
*C12P 21/06* (2006.01)

(86) International application number:
**PCT/US2009/069868**

(87) International publication number:
**WO 2010/078462 (08.07.2010 Gazette 2010/27)**

(54) **PROTEIN HYDROLYSATE COMPOSITIONS**

PROTEINHYDROLYSATZUSAMMENSETZUNGEN

COMPOSITIONS D'HYDROLYSAT DE PROTÉINE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **31.12.2008 US 141941 P**

(43) Date of publication of application:
**09.11.2011 Bulletin 2011/45**

(73) Proprietors:
• **Solae, LLC
St. Louis, MO 63110 (US)**
• **Novozymes A/S
2880 Bagsvaerd (DK)**

(72) Inventors:
• **HWANG, Der-Chyan
Chesterfield
Missouri 63017 (US)**

• **SHAH, Naina K.
Manchester
Missouri 63021 (US)**
• **KERR, Phillip S.
Wildwood
Missouri 63005 (US)**
• **WONG, Theodore M.
Ballwin
Missouri 63017 (US)**
• **LYNGLEV, Gitte Budolfsen
DK-2000 Frederiksberg (DK)**

(74) Representative: **D Young & Co LLP
120 Holborn
London EC1N 2DY (GB)**

(56) References cited:
**EP-A1- 1 512 328     EP-A2- 0 976 331
WO-A1-91/13554     WO-A1-2007/103753
WO-A2-2008/131008     US-A- 4 100 024
US-A- 6 036 983     US-B1- 6 171 640**

SEQUENCE LISTING

<110> Solae, LLC Hwang, Der-Chyan Lynglev, Gitte Budolfsen Shah, Naina K. Kerr, Phillip S. Wong, Theodore M.

<120> Protein Hydrolysate Compositions

<130> SP-1563 PCT

<150> 61/141,941 <151> 2008-12-31

<160> 38

<170> PatentIn version 3.5

<210> 1
<211> 315
<212> PRT
<213> Bacillus licheniformis

<400> 1

```
Met Val Ser Lys Lys Ser Val Lys Arg Gly Leu Ile Thr Gly Leu Ile
1               5               10                  15

Gly Ile Ser Ile Tyr Ser Leu Gly Met His Pro Ala Gln Ala Ala Pro
            20              25                  30

Ser Pro His Thr Pro Val Ser Ser Asp Pro Ser Tyr Lys Ala Glu Thr
        35              40                  45

Ser Val Thr Tyr Asp Pro Asn Ile Lys Ser Asp Gln Tyr Gly Leu Tyr
        50              55                  60

Ser Lys Ala Phe Thr Gly Thr Gly Lys Val Asn Glu Thr Lys Glu Lys
65              70              75                  80

Ala Glu Lys Lys Ser Pro Ala Lys Ala Pro Tyr Ser Ile Lys Ser Val
                85              90                  95

Ile Gly Ser Asp Asp Arg Thr Arg Val Thr Asn Thr Thr Ala Tyr Pro
            100             105                 110

Tyr Arg Ala Ile Val His Ile Ser Ser Ile Gly Ser Cys Thr Gly Trp
        115             120                 125

Met Ile Gly Pro Lys Thr Val Ala Thr Ala Gly His Cys Ile Tyr Asp
        130             135                 140

Thr Ser Ser Gly Ser Phe Ala Gly Thr Ala Thr Val Ser Pro Gly Arg
```

                    145                    150                    155                    160

Asn Gly Thr Ser Tyr Pro Tyr Gly Ser Val Lys Ser Thr Arg Tyr Phe
                    165                    170                    175

Ile Pro Ser Gly Trp Arg Ser Gly Asn Thr Asn Tyr Asp Tyr Gly Ala
                    180                    185                    190

Ile Glu Leu Ser Glu Pro Ile Gly Asn Thr Val Gly Tyr Phe Gly Tyr
                    195                    200                    205

Ser Tyr Thr Thr Ser Ser Leu Val Gly Thr Thr Val Thr Ile Ser Gly
        210                    215                    220

Tyr Pro Gly Asp Lys Thr Ala Gly Thr Gln Trp Gln His Ser Gly Pro
225                    230                    235                    240

Ile Ala Ile Ser Glu Thr Tyr Lys Leu Gln Tyr Ala Met Asp Thr Tyr
                    245                    250                    255

Gly Gly Gln Ser Gly Ser Pro Val Phe Glu Gln Ser Ser Ser Arg Thr
            260                    265                    270

Asn Cys Ser Gly Pro Cys Ser Leu Ala Val His Thr Asn Gly Val Tyr
            275                    280                    285

Gly Gly Ser Ser Tyr Asn Arg Gly Thr Arg Ile Thr Lys Glu Val Phe
        290                    295                    300

Asp Asn Leu Thr Asn Trp Lys Asn Ser Ala Gln
305                    310                    315

<210> 2
<211> 9
<212> PRT
<213> Glycine max

<400> 2

                    Asn Lys Val Leu Phe Ser Arg Glu Glu
                    1                    5

<210> 3
<211> 10
<212> PRT
<213> Glycine max

<400> 3

```
Ile Asn Lys Val Leu Phe Ser Arg Glu Glu
1               5                   10
```

<211> 9
<212> PRT
<213> Glycine max

<400> 4

```
Ala Ser Tyr Asp Thr Lys Phe Glu Glu
1               5
```

<210> 5
<211> 8
<212> PRT
<213> Glycine max

<400> 5

```
Lys Leu Leu Lys Asn Gln Arg Glu
1               5
```

<210> 6
<211> 5
<212> PRT
<213> Glycine max

<400> 6

```
Leu Leu Leu Pro Asn
1               5
```

<210> 7
<211> 4
<212> PRT
<213> Glycine max

<400> 7

```
Phe Lys Asn Gln
1
```

<210> 8
<211> 7
<212> PRT
<213> Glycine max

<400> 8

```
Leu Lys Val Arg Glu Asp Glu
1               5
```

<210> 9
<211> 5
<212> PRT
<213> Glycine max

<400> 9

```
Gly Val Ile Val Glu
1               5
```

<211> 4
<212> PRT
<213> Glycine max

<400> 10

```
Arg Ile Val Gln
1
```

<210> 11
<211> 8
<212> PRT
<213> Glycine max

<400> 11

```
Lys Leu Gln Ser Pro Asp Asp Glu
1               5
```

<210> 12
<211> 6
<212> PRT
<213> Glycine max

<400> 12

```
Asn Ser Gln His Pro Glu
1               5
```

<210> 13
<211> 11
<212> PRT
<213> Glycine max

<400> 13

```
Phe Ser Ser Arg Glu Gln Pro Gln Gln Asn Glu
1               5                   10
```

<210> 14
<211> 7
<212> PRT
<213> Glycine max

<400> 14

```
                          Phe Leu Lys Tyr Gln Gln Glu
                          1               5
```

<210> 15
<211> 12
<212> PRT
<213> Glycine max

<400> 15

```
              Ala Gly Val Lys Ala Pro Ser Ile Tyr His Ser Glu
              1               5                   10
```

<211> 12
<212> PRT
<213> Glycine max

<400> 16

```
              Asn Leu Ala Ala Ala Ala Lys His His Leu Ala Glu
              1               5                   10
```

<210> 17
<211> 6
<212> PRT
<213> Glycine max

<400> 17

```
                          Leu Val Gly Leu Lys Glu
                          1               5
```

<210> 18
<211> 5
<212> PRT
<213> Glycine max

<400> 18

```
                          Asn Phe Leu Ala Gly
                          1               5
```

<210> 19
<211> 5
<212> PRT
<213> Glycine max

<400> 19

```
                              Gln Pro Arg Gln Glu
                              1               5
```

<210> 20
<211> 5
<212> PRT
<213> Glycine max

<400> 20

```
                              Ser Val Ile Val Glu
                              1               5
```

<210> 21
<211> 11
<212> PRT
<213> Glycine max

<400> 21

```
                    Phe Val Asp Ala Gln Pro Gln Gln Lys Glu Glu
                    1               5                   10
```

<211> 14
<212> PRT
<213> Glycine max

<400> 22

```
                Ser Tyr Phe Val Asp Ala Gln Pro Gln Gln Lys Glu Glu Glu
                1               5                   10
```

<210> 23
<211> 9
<212> PRT
<213> Glycine max

<400> 23

```
                    Lys Asp Asn Val Val Arg Gln Ile Glu
                    1               5
```

<210> 24
<211> 6
<212> PRT
<213> Glycine max

<400> 24

```
                        Leu Val Gly Ile Lys Glu
                        1               5
```

<210> 25
<211> 6
<212> PRT
<213> Glycine max

<400> 25

```
                        Gln Pro Gln Gln Lys Glu
                        1               5
```

<210> 26
<211> 5
<212> PRT
<213> Glycine max

<400> 26

```
                          Arg Leu Leu Lys Lys
                          1               5
```

<210> 27
<211> 8
<212> PRT
<213> Glycine max

<400> 27

```
                        Arg Lys Gln Ile Val Thr Val Glu
                        1               5
```

<211> 7
<212> PRT
<213> Glycine max

<400> 28

```
                        Gln Gln Glu Glu Glu Glu Glu
                        1               5
```

<210> 29
<211> 14
<212> PRT
<213> Glycine max

<400> 29

```
                    Val Gly Gln Asp Ile Gln Ser Lys Ala Gln Asp Thr Arg Glu
                    1               5                   10
```

<210> 30
<211> 10
<212> PRT
<213> Glycine max

<400> 30

```
Ala Ala Ala Ala Lys His His Leu Ala Glu
1               5                   10
```

<210> 31
<211> 6
<212> PRT
<213> Glycine max

<400> 31

```
Gly Leu Thr Val Asp Glu
1               5
```

<210> 32
<211> 4
<212> PRT
<213> Glycine max

<400> 32

```
Phe Leu Trp Gly
1
```

<210> 33
<211> 8
<212> PRT
<213> Glycine max

<400> 33

```
Ile Thr Ser Ser Lys Phe Asn Glu
1               5
```

<211> 4
<212> PRT
<213> Glycine max

<400> 34

```
Lys Phe Asn Glu
1
```

<210> 35
<211> 5
<212> PRT
<213> Glycine max

<400> 35

```
                          His Ile Met Glu Lys
                          1               5
```

<210> 36
<211> 4
<212> PRT
<213> Glycine max

<400> 36

```
                          Lys Met Glu Lys
                          1
```

<210> 37
<211> 4
<212> PRT
<213> Glycine max

<400> 37

```
                          Lys Met Glu Glu
                          1
```

<210> 38
<211> 4
<212> PRT
<213> Glycine max

<400> 38

```
                          Lys Phe Leu Gln
                          1
```

Description

## CROSS REFERENCE TO RELATED APPLICATION

This application claims the priority of US provisional application number 61/141,941 filed December 31, 2008, which is hereby incorporated by reference in its entirety.

## FIELD OF THE INVENTION

The present invention relates to protein hydrolysate compositions that include the hydrophobic core region of ß-conglycinin, have a higher whiteness index, and unique viscoelastic properties that include thickening at lower temperatures, processes for making the protein hydrolysate compositions, and food products comprising the protein hydrolysate compositions.

## BACKGROUND OF THE INVENTION

Increased protein consumption worldwide along with limited availability and increased expense of animal-based proteins and dairy proteins has led to increased interest in vegetable proteins such as soy protein.

While soy is an excellent source of protein, it tends to have a grey color making it unsuitable for use in some food products. The grey or light beige color of soy proteins often pose a challenge for the use of soy protein in various products like beverages, whole muscle applications, and cheeses. Consumers do not always appreciate the grey or light beige color. What is needed, therefore, is a soy protein product having increased whiteness. Further, soy protein typically requires high heat and high solids to coagulate; therefore it has been difficult to make a soy cheese with properties similar to dairy based cheese. It would be helpful to have a soy protein that coagulates when minimal heat is applied.

WO 91/13554 A1 relates to a protein hydrolysate which consists essentially of peptides with a C-terminal glutamic or aspartic acid residue as a result of specific hydrolysis of proteins at Glu and/or Asp bonds with an enzyme preparation.

## SUMMARY OF THE INVENTION

Among the various aspects of the present invention is the provision of a protein hydrolysate composition that includes an enriched hydrophobic core region (47 kDa fragment) of ß-conglycinin. The protein hydrolysate composition comprises a mixture of polypeptides enriched in a 47 kiloDalton (kDa) fragment, wherein at least about 10% to about 66% of the polypeptides by weight are the 47 kDa fragment.

The invention further discloses a protein fraction comprising an enriched hydrophobic ß-conglycinin core region. ß-conglycinin and it's derived peptides are known to reduce cholesterol in mammals. (Adams et al. 2004, Lovati et.al. 2000, Cho et al., 2008).

Another aspect of the invention encompasses a process for preparing a protein hydrolysate composition. The process comprises contacting a protein material with an endopeptidase that cleaves the protein material into mixture of polypeptides enriched in a 47 kDa fragment. A further aspect discloses a process for preparing a protein fraction.

One aspect of the invention encompasses a process for producing a hydrophobic ß-conglycinin core region enriched protein product, wherein the hydrophilic extension region of the α' and a subunits of ß-conglycinin are hydrolyzed from ß-conglycinin by using an enzyme that cleaves proteins at the C-terminus of glutamic acid and aspartic acid to yield the hydrophobic ß-conglycin core region enriched protein product, wherein at least about 10% to about 66% of the polypeptides by weight are the 47 kDa fragment.

A further aspect of the invention provides food products comprising a protein hydrolysate composition. The protein hydrolysate composition comprises a mixture of polypeptides enriched in a 47 kDa fragment, wherein at least about 10% to about 66% of the polypeptides by weight are the 47 kDa fragment.

Other aspects and features of the invention will be in part apparent and in part pointed out hereinafter.

## REFERENCE TO COLOR FIGURES

The application contains at least one photograph executed in color. Copies of this patent application publication with color photographs will be provided by the Office upon request and payment of the necessary fee.

## DESCRIPTION OF THE FIGURES

**Figure 1** is a SDS-PAGE of an isolated soy protein hydrolyzed using GE. Lane 1 is a standard soy protein isolate SUPRO® 500E, lane 2 -control unhydrolyzed isolate, lane 3- isolate hydrolyzed with serine protease (also called glutamyl endopeptidase (GE)) from *Bacillus lichenformis,* 12mg GE/kg of isolate, lane 4-isolate hydrolyzed with 24 mg GE/kg of isolate, lane 5- isolate hydrolyzed with 48 mg GE/kg of isolate and lane 6 is molecular weight standard.

**Figure 2** is an image of a Coomassie-stained SDS-polyacrylamide gel (SDS-PAGE) of commodity soy protein in flours with and without GE treatment. Lane 1 contains the commodity soy protein control, without GE treatment Lane 2 contains commodity soy protein treated with 2.4 mg GE/Kg protein. Lane 3 contains commodity soy protein treated with 4.8 mg GE/Kg protein. The $\alpha$' and $\alpha$ subunits of ß-conglycinin are indicated on the left and the 47kDa region is indicated on the left.

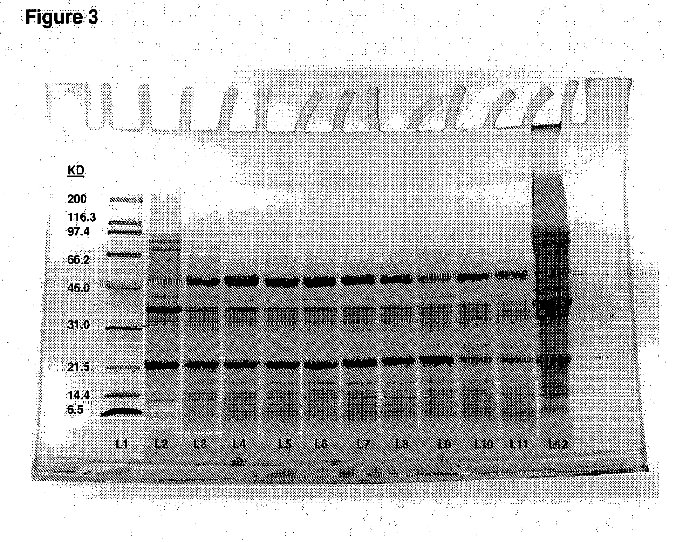

**Figure 3** is a SDS-PAGE of an acid precipitated soy protein curd hydrolyzed using GE. Lane 1 shows molecular weight standards, lane 2 is control curd slurry without hydrolysis, lanes 3, 4 ,and 5 contain samples hydrolyzed for 30 minutes with GE concentration of 24, 48 and 96 mg GE/kg of curd slurry solids respectively, lanes 6, 7 ,and 8 contain samples hydrolyzed for 60 minutes with GE concentration of 24, 48 and 96 mg GE/kg of curd slurry solids respectively, lanes 9, 10, and 11 contain samples hydrolyzed for 120 minutes with GE concentration of 24, 48 and 96 mg GE/kg of curd slurry solids respectively, lane 12 contains standard soy protein isolate SUPRO® 500E.

**Figure 4** shows a comparison of the whiteness index of GE treated isolated soy protein hydrolysates versus another serine protease, serine protease (SP) from *Nocardiopsis prasina* treated isolated soy protein hydrolysates.

**Figure 5** shows the enrichment of 47 kDa polypeptide from GE hydrolysate by NaCl treatment. I: indicates the original sample with treatment of 4.8 mg GE/Kg protein; II: describes the process of dissolving protein in 2% NaCl and mix at 75°C for 30 min; III: describes the process of centrifuging the NaCl treated sample at 3,000 x G for 10 min; IV: indicates the supernatant fraction which mainly consists of 11 S and beta subunit of 7S enriched fraction; V: describes the precipitate fraction which mainly consists of 47 kDa polypeptide enriched fraction; VI: indicates the intact soy protein.

**Figure 6** shows an anionic exchange chromatography separation of the 47 kDa fragments from other protein components in GE treated soy protein along with SDS-PAGE analysis of each fraction (1-10) collected from the chromatography separation. Gel A is from the control protein and Gel B is from 4.8 mg GE/Kg protein hydrolyzed sample. Fraction 1 shows the protein in the unbound fraction in the chromatography. The major protein band in fraction 1 of Gel B is the 47 kDa polypeptide.

**Figure 7** shows the Thermo Hydration Ratio (THR) values for control, 2.4 mg GE/ Kg protein, and 4.8 mg GE/Kg protein, i.e. examples 1-3 of the current invention.

**Figure 8** shows the Rapid Visco Analyzer (RVA) test results of the GE modified soy protein.

**Figure 9** indicates potential GE cleavage sites (underlined residues) for the a subunit of $\beta$-conglycinin, 47 kDa section is shown in bold.

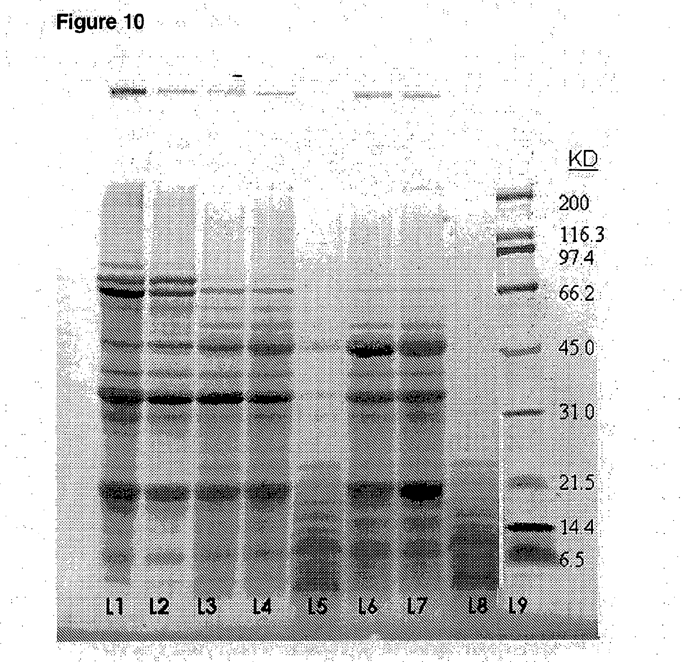

**Figure 10** is a SDS-PAGE of GE treated hydrolysates from a large scale preparation. Lane 1 contains standard soy protein isolate SUPRO® 500E. Lane 2 control isolate without enzyme, Lane 3 is sample hydrolyzed with 6mg GE/kg protein of sample on dry matter basis, lane 4 is the precipitate from subjecting sample hydrolyzed with 6mg GE/kg protein to centrifugation, lane 5 is supernatant from subjecting sample hydrolyzed with 6mg GE/kg protein to centrifugation. Lane 6 is sample hydrolyzed with 12mg GE/kg protein of sample on dry matter basis, lane-7 is the precipitate from subjecting sample hydrolyzed with 12mg GE/kg protein to centrifugation, lane 8 is supernatant from subjecting sample hydrolyzed with 12mg GE/kg protein to centrifugation. Lane 9 is molecular weight standards.

**Figure 11** is a color photograph of gels made from GE treated soy protein hydrolysates with 6 and 12 mg GE/kg protein enzyme dose. Gels were made from hydrolysates and hydrolysates UF where hydrolysates were subjected to precipitation at pH 4.5 to remove shorter fragments.

**Figure 12** shows the comparison of SQS sensory data of GE treated sample versus control.

**Figure 13** shows a comparison of the whiteness index of GE treated acid curd versus SP treated acid curd.

## DETAILED DESCRIPTION OF THE INVENTION

The present invention provides protein hydrolysate compositions, processes for making protein hydrolysate compositions, and food products comprising protein hydrolysate compositions. It has been discovered that digestion of a soy protein with an endopeptidase that specifically cleaves the soy protein material on the carboxyl terminal (C-terminus) side of glutamic acid or aspartic acid results in compositions comprising polypeptide fragments of about 47 kDa. The 47 kDa protein fragment is comprised of the hydrophobic ß-conglycinin core region of the ß-conglycinin. The hydrophobic $\beta$-conglycinin core region of the ß-conglycinin is comprised of a and $\alpha$' subunits which are typically 47 kDa.

The β subunit of β-conglycinin is about 47-50 kDa. The composition of the current invention includes about 10% to about 66% 47 kDa fragment. These protein hydrolysate compositions have improved whiteness indexes and unique viscoelastic properties compared to the starting soy protein material making them ideal for yogurts, cheese, puddings, and whipped food products.

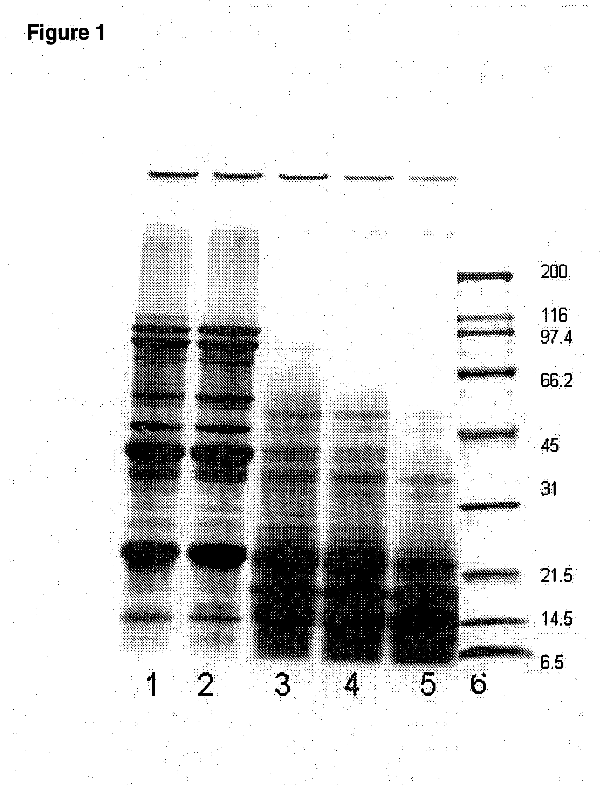

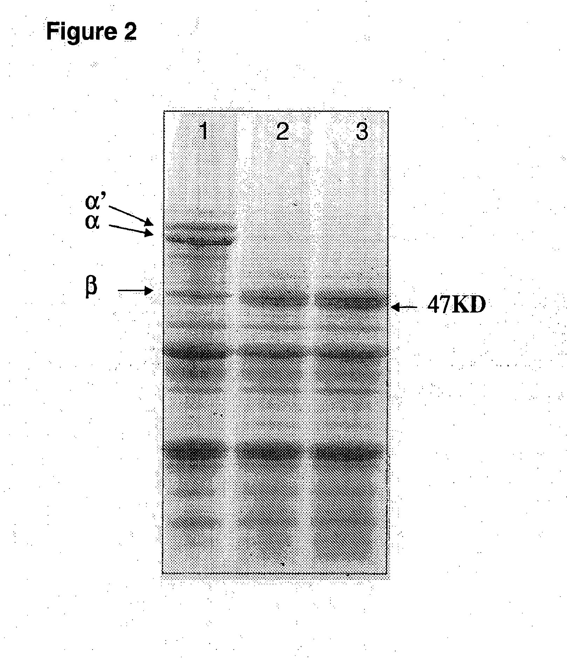

Serine Protease, also called glutamyl endopeptidase (termed "GE") from *Bacillus lichenformis* (UNIPROT: P80057 as disclosed and characterized in US Patent Nos. 4,266,031, 5,874,278, and 5,459,064 and International Application Nos. WO 01/16285, WO 92/13964, WO 91/13553, and WO 91/13554, each of which is incorporated by reference in its entirety) cleaves proteins at the C-terminal of glutamic acid and aspartic acid residues. By modifying the substrate, different hydrolysis outcomes were achieved as a result of the enzymatic reaction at the same enzyme and substrate concentrations. When the GE reaction was carried out on spray dried soy protein isolates, the reaction was more random, generating peptide fragments of various molecular weights as was evident from the SDS-PAGE (Figure 1), however when the reaction was carried out on the soy protein slurries made from the soy flakes by isoelectric precipitation of the proteins, the proteins were in a more native form and the hydrolysis pattern differed greatly. The hydrolysis resulted in a more selective cleavage and accumulation of 47 kDa protein fragment, it also appeared to be very specific to cleavage of ß-conglycinin and bands pertaining to glycinin seemed unchanged as a result of GE hydrolysis based on SDS-PAGE data (**Figures 2 and 3).** This technology allows for the generation of material with enriched ß-conglycinin core region fractions on a large scale.

*I.* **Process** *for Preparing a Protein Hydrolysate Composition*

One aspect of the present invention provides a process for preparing a protein hydrolysate composition comprising a mixture of polypeptides enriched in a 47 kDa fragment. The process comprises contacting a protein material with an endopeptidase that specifically cleaves the protein material on the carboxyl terminal side of glutamic acid or aspartic acid, thereby creating a mixture of polypeptides enriched in a 47 kDa fragment.

**a. hydrolytic cleavage**

The process comprises cleaving the protein material into a mixture of smaller sized polypeptides. In general, the protein material is contacted with an endopeptidase to form a hydrolysate. The hydrolysate may be cleaved further using an exopeptidase.

*i. protein material*

Non-limiting examples of suitable protein materials include soy, amaranth, arrowroot, buckwheat, canola, cassava, channa (garbonzo), corn, legume, lentils, lupin, millet, oat, pea, rye sorghum, sunflower, tapioca, triticale, dairy, and combinations thereof. In one embodiment, the protein material may be a soy protein material. A variety of soy protein materials may be used in the process of the invention to generate a soy protein hydrolysate. In general, the soy protein material may be derived from whole soybeans in accordance with methods known in the art. The whole soybeans may be commodity soybeans (i.e., non genetically modified soybeans), genetically modified soybeans, and combinations thereof. Genetically modified soybeans include soybeans with modified protein, oil, or carbohydrate compositions, such as high ß-conglycinin soybeans and high oleic soybeans. Suitable examples of soy protein material include soy extract, soy curd, soy flour, isolated soy protein, soy protein concentrate, soymilk, soymilk powder, and mixtures thereof.

In one embodiment, the soy protein material used in the process may be an isolated soy protein (also called ISP or soy protein isolate). In general, isolated soy proteins have a protein content of at least about 90% soy protein on a moisture-free basis. The isolated soy protein may comprise intact soy proteins or it may comprise partially hydrolyzed soy proteins. The isolated soy protein may have a high content of storage protein subunits such as 7S, 11S, 2S, and 15S. Non-limiting examples of isolated soy proteins that may be used in the present invention are commercially available, for example, from Solae, LLC (St. Louis, MO), and include SUPRO® 500E, SUPRO® 620, SUPRO® 710, and SUPRO® EX 33 isolated soy proteins.

In another embodiment, the soy protein material may be a soy protein concentrate (SPC), which has a protein content of about 65% to less than about 90% on a moisture-free basis. Examples of suitable soy protein concentrates useful in the invention include ALPHA® DSP-C, Procon™, ALPHA® 12 and ALPHA® 5800 which are commercially available from Solae, LLC (St. Louis, MO). Alternatively, soy protein concentrate may be blended with the isolated soy protein to substitute for a portion of the isolated soy protein as a source of soy protein material.

In yet another embodiment, the soy protein material may bye soy flour, which has a protein content of about 49% to about 65% on a moisture-free basis. The soy flour may be defatted soy flour, partially defatted soy flour, or full fat soy flour. Alternatively, soy flour may be blended with isolated soy protein or soy protein concentrate. Any combination of soy flour, isolated soy protein, or soy protein concentrate will work. One skilled in the art can combine these ingredients

depending on the desired attributes of the end use product.

When soy flour is used, the starting material is typically a defatted soy flour or flakes. Full fat soybeans contain approximately 40% protein by weight and approximately 20% oil by weight. These whole full fat soybeans may be defatted through conventional processes when a defatted soy flour or flakes form the starting protein material. For example, the soybean may be cleaned, dehulled, cracked, passed through a series of flaking rolls and then subjected to solvent extraction by use of hexane or other appropriate solvents to extract the oil and produce defatted flakes. The defatted flakes may be ground to produce a soy flour. Although the process is yet to be employed with full fat soy flour, it is believed that full fat soy flour may also serve as a protein source. However, where full fat soy flour is processed, it is most likely necessary to use a separation step, such as three stage centrifugation to remove oil.

In a further embodiment, the soy protein material may be soymilk. Alternatively, soymilk may be blended with isolated soy protein, soy protein concentrate, or soy flour. Any combination of soy flour, isolated soy protein, or soy protein concentrate will work. One skilled in the art can combine these ingredients, depending on the desired attributes of the end use product.

In an alternate embodiment, the soy protein material may be soymilk powder. Alternatively, soymilk powder may be blended with isolated soy protein, soy protein concentrate, or soy flour. Any combination of soy flour, isolated soy protein, or soy concentrate will work. One skilled in the art can combine these ingredients depending on the desired attributes of the end use product.

In a further alternate embodiment, the soy protein material may be material that has been separated into four major storage protein fractions or subunits (7S, 11S. 2S, and 15S) on the basis of sedimentation in a centrifuge. In general, the 11S fraction is highly enriched in glycinin and the 7S fraction is highly enriched in ß-conglycinin.

In general, the starting soy protein material typically comprises a mixture of proteins having a range of sizes. In one embodiment, the proteins in the soy material may range in size from about 1000 Daltons to about 500,000 Daltons. In another embodiment, the proteins in the soy material may range in size from about 3000 Daltons to about 100,000 Daltons.

In another embodiment, the protein material may be derived from a plant other than soy. By way of non-limiting example, suitable plants include amaranth, arrowroot, barley, buckwheat, canola, cassava, channa (garbanzo), legumes, lentils, lupin, maize, millet, oat, pea, potato, rice, rye, sorghum, sunflower, tapioca, triticale, wheat, and mixtures thereof. Especially preferred plant proteins include barley, canola, lupin, maize, oat, pea, potato, rice, wheat, and combinations thereof. In one embodiment, the plant protein material may be canola meal, canola protein isolate, canola protein concentrate, and combinations thereof. In another embodiment, the plant protein material may be maize or corn protein powder, maize or corn protein concentrate, maize or corn protein isolate, maize or corn germ, maize or corn gluten, maize or corn gluten meal, maize or corn flour, zein protein, and combinations thereof. In still another embodiment, the plant protein material may be barley powder, barley protein concentrate, barley protein isolate, barley meal, barley flour, and combinations thereof. In an alternate embodiment, the plant protein material may be lupin flour, lupin protein isolate, lupin protein concentrate, and combinations thereof. In another alternate embodiment, the plant protein material may be oatmeal, oat flour, oat protein flour, oat protein isolate, oat protein concentrate, and combinations thereof. In yet another embodiment, the plant protein material may be pea floor, pea protein isolate, pea protein concentrate, and combinations thereof. In still another embodiment, the plant protein material may be potato protein powder, potato protein isolate, potato protein concentrate, potato flour, and combinations thereof In a further embodiment, the plant protein material may be rice flour, rice meal, rice protein powder, rice protein isolate, rice protein concentrate, and combinations thereof. In another alternate embodiment, the plant protein material may be wheat protein powder, wheat gluten, wheat germ, wheat flour, wheat protein isolate, wheat protein concentrate, solubilized wheat proteins, and combinations thereof.

In another embodiment, the protein material may be derived from dairy. Non-limiting examples of suitable dairy proteins include non-fat dried milk powder, milk protein isolate, milk protein concentrate, acid casein, caseinate (e.g. sodium caseinate and calcium caseinate) casein protein isolate, casein protein concentrate, whey protein concentrate, whey protein isolate, and combinations thereof. The milk protein material may be derived from cows, goats, sheep, donkeys, camels, camelids, yaks, and water buffalos.

It is also envisioned that combinations of soy protein material and at least one other protein material may be used in the process of the invention. That is, a protein hydrolysate composition may be prepared from a combination of a soy protein material and at least one other protein Material. In one embodiment, a protein hydrolysate composition may be prepared from a combination of a soy protein material and one other protein material selected from the group consisting of barley, canola, lupin, maize, oat, pea, potato, rice, wheat, and dairy. In another embodiment, a protein hydrolysate composition may be prepared from a combination of a soy protein material and two other protein materials selected from the group consisting of barley, canola, lupin, maize, oat, pea, potato, rice, wheat, and dairy. In further embodiments, a protein hydrolysate composition may be prepared from a combination of a soy protein material and three or more other protein materials selected from the group consisting of barley, canola, lupin, maize, oat pea potato, rice, wheat, and dairy. Non-limiting combinations include soy protein and dairy protein, soy protein and canola protein, soy protein and wheat protein, soy protein and corn protein, soy protein and rice protein, soy protein and pea protein, soy protein and lupine protein.

Further, combinations of the various protein materials apart from and including the soy protein are possible.

## b. **protein slurry**

The concentrations of the soy protein material and the other protein material used in combination can and will vary. The amount of soy protein material may range from about 1 % to about 99% of the total protein used in the combination. In one embodiment, the amount of soy protein material may range from about 1% to about 20% of the total protein used in combination. In another embodiment, the amount of soy protein material may range from about 20% to about 40% of the total protein used in combination. In still another embodiment, the amount of soy protein material may range from about 40% to about 80% of the total protein used in combination. In a further embodiment, the amount of soy protein material may range from about 80% to about 99% of the total protein used in combination. Likewise, the amount of the (at least one) other protein material may range from about 1 % to about 99% of the total protein used in combination. In one embodiment, the amount of other protein material may range from about 1% to about 20% of the total protein used in combination. In another embodiment, the amount of other protein material may range from about 20% to about 40% of the total protein used in combination. In still another embodiment, the amount of other protein material may range from about 40% to about 80% of the total protein used in combination. In a further embodiment, the amount of other protein material may range from about 80% to about 99% of the total protein used in combination.

In the process of the invention, the protein material is typically mixed or dispersed in water to form a slurry comprising about 1% to about 25% protein (as is) by weight. In one embodiment, the slurry may comprise about 1 % to about 5% protein (as is) by weight. In another embodiment, the slurry may comprise about 6% to about 10% protein (as is) by weight. In a further embodiment, the slurry may comprise about 11 % to about 15% protein (as is) by weight. In still another embodiment, the slurry may comprise about 16% to about 25% protein (as is) by weight.

After the protein material is dispersed in water, the slurry of protein material may be heated from about 70°C to about 90°C for about 2 minutes to about 20 minutes to inactivate putative endogenous protease inhibitors. The pH and the temperature of the protein slurry can be adjusted so as to optimize the hydrolysis reaction, and to ensure that the endopeptidase used in the hydrolysis reaction functions near its optimal activity level. The pH of the protein slurry may be adjusted and monitored according to methods generally known in the art. The pH of the protein slurry may be adjusted and maintained at a value from about 5.0 to about 11.0. However, in one embodiment, the pH of the protein slurry is not adjusted. The pH is maintained at from about 6.3 to about 6.8. The enzyme treated protein slurry is then incubated for 30 minutes at a pH from about 6.3 to about 6.8 and then the pH is adjusted to 7.0. In one embodiment, the pH of the protein slurry may be adjusted and maintained at from about 7.0 to about 8.0. In another embodiment, the pH of the protein slurry may be adjusted and maintained at from about 8.0 to about 9.0. In yet another embodiment, the pH of the protein slurry may be adjusted and maintained at from about 9.0 to about 10.0. In a preferred embodiment, the pH of the protein slurry may be adjusted and maintained at about 8.0. The temperature of the protein slurry is preferably adjusted and mantained at from about 30°C to about 70°C during the hydrolysis reaction in accordance with methods known in the art. In general, temperatures above or below this range may inactivate the endopeptidase. In one embodiment, the temperature of the protein slurry may be adjusted and maintained at from about 40°C to about 60°C during the hydrolysis reaction in accordance with methods known in the art.

### i. endopeptidase

The hydrolysis reaction is generally initiated by adding an endopeptidase to the slurry of protein material to form a reaction mixture. Reaction of the endopeptidase with the protein material leads to hydrolysis of the protein material into smaller polypeptides. This endopeptidase will selectively cleave proteins on the carboxyl terminal side of either glutamic acid or aspartic acid. The endopeptidase is a serine protease, specifically GE. GE has an optimal activity at a pH from about 6.0 to about 11.0 and at a temperature from about 30°C to about 70°C, with the optimum temperature being about 50°C at pH 9.0.

The endopeptidase can be an enzyme of microbial origin. The use of microbial enzymes rather than animal or plant enzymes is advantageous in that microbial enzymes exhibit a broad spectrum of characteristics (pH optima, temperature; etc.) and may be consistently obtainable in relatively large quantities. A non-limiting example of suitable endopeptidases include GE a serine protease from *Bacillus licheniformis* (UNIPROT: P80057) as previously discussed. GE cleaves the peptide bond on the carboxyl terminal side of glutamic acid or aspartic acid.

In one embodiment, the endopeptidase may be a serine protease having an amino acid sequence that is at least 80% identical to SEQ ID NO:1 shown in Table 1, and has protease activity. In another embodiment, the endopeptidase may be a serine protease having an amino acid sequence that is at least 85% identical to SEQ ID NO:1 and has protease activity. In still another embodiment, the endopeptidase may be a serine protease having an amino acid sequence that is at least 90% identical to SEQ ID NO:1 and has protease activity. In an alternate embodiment, the endopeptidase may be a serine protease having an amino acid sequence that is at least 95% identical to SEQ ID NO:1 and has protease activity. In another alternate embodiment, the endopeptidase may be a serine protease having an amino acid sequence that is at least 97% identical to SEQ ID NO:1 and has protease activity. In a further embodiment, the endopeptidase may be a serine protease having an amino acid sequence that is at least 99% identical to SEQ ID NO:1 and has protease activity.

**Table 1**

| SEQ ID NO:1 | MVSKKSVKRGLITGLIGISIYSLGMHPAQAAPSPHTPVSSDPSYKAETSVTYDPNIKSD QYGLYSKAFTGTGKVNETKEKAEKKSPAKAPYSIKSVIGSDDRTRVTNTTAYPYRAIV HISSIGSCTGWMIGPKTVATAGHCIYDTSSGSFAGTATVSPGRNGTSYPYGSVKSTR YFIPSGWRSGNTNYDYGAIELSEPIGNTVGYFGYSYTTSSLVGTTVTISGYPGDKTAG TQWQHSGPIAISETYKLQYAMDTYGGQSGSPVFEQSSSRTNCSGPCSLAVHTNGVY GGSSYNRGTRITKEVFDNLTNWKNSAQ |
|---|---|

The degree of sequence identity between two amino acid sequences may be determined using the BLASTp algorithm of Karlin and Altschul (Proc. Natl. Acad. Sci. USA 87:2264-2268, 1990). The percentage of sequence identity is determined by comparing two optimally aligned sequences over a comparison window, wherein the portion of the amino acid sequence in the comparison window may comprise additions or deletions (i.e., gaps) as compared to the reference sequence (which does not comprise additions or deletions) for optimal alignment of the two sequences. The percentage is calculated by determining the number of positions at which an identical amino acid occurs in both sequences to yield the number of matched positions, dividing the number of matched positions by the total number of positions in the window of comparison and multiplying the result by 100 to yield the percentage of sequence identity.

A skilled practitioner will understand that an amino acid residue may be substituted with another amino acid residue having a similar side chain without affecting the function of the polypeptide. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acid substitution groups include: valine-leucine-isoleucine, phenylalanine-tyrosine, lysine-arginine, alanine-valine, and asparagine-glutamine. Thus, the endopeptidase may have at least one conservative amino acid substitution with respect to SEQ ID NO:1. In one embodiment, the endopeptidase may have about 35 conservative amino acid substitutions with respect to SEQ ID NO:1. In another embodiment, the endopeptidase may have about 25 conservative amino acid substitutions with respect to SEQ ID NO:1. In yet another embodiment, the endopeptidase may have about 15 conservative amino acid substitutions with respect to SEQ ID NO:1. In still embodiment, the endopeptidase may have about 10 conservative amino acid substitutions with respect to SEQ ID NO: 1. In yet another embodiment, the endopeptidase may have about 5 conservative amino acid substitutions with respect to SEQ ID NO:1. In a further embodiment, the endopeptidase may have about one conservative amino acid substitutions with respect to SEQ ID NO:1.

The fragment of any of these sequences having protease activity may be the amino acid sequence of the active enzyme, e.g. after processing, such as after any signal peptide and/or propeptide has been cleaved off.

The endopeptidase may also be an enzyme of mammalian origin, e.g. bovine or porcine origin.

Various combinations of protein material and endopeptidase are presented in Table 2.

**Table 2**

| Protein Material | Endopeptidase |
|---|---|
| Soy | GE |
| Canola | GE |
| Chickpea | GE |
| Corn (Maize) | GE |
| Fava bean | GE |
| Legume | GE |
| Wheat | GE |
| Rice | GE |

(continued)

| Protein Material | Endopeptidase |
| --- | --- |
| Pea | GE |
| Lupin | GE |
| Dairy | GE |

The amount of endopeptidase added to the protein slurry can and will vary depending upon the source of the protein material, the desired degree of hydrolysis, and the duration of the hydrolysis reaction. The amount may range from about 1 mg to about 5000 mg of endopeptidase per kilogram of protein material. In one embodiment, the amount may range from 1 mg to about 20 mg of endopeptidase per kilogram of protein material. In another embodiment, the amount may range from 20 mg to about 1000 mg of endopeptidase per kilogram of protein material. In yet another embodiment, the amount may range from about 1000 mg to about 5000 mg of endopeptidase per kilogram of protein material.

As will be appreciated by a skilled artisan, the duration of the hydrolysis reaction can and will vary, depending upon the concentration of the endopeptidase and the desired degree of hydrolysis, for example. Generally speaking, the duration of the hydrolysis reaction may range from a few minutes to many hours, such as, from about 5 minutes to about 5 hours. In preferred embodiment, the duration of the reaction may be about 30 minutes.

To end the hydrolysis reaction, the reaction mixture may be heated to a temperature that is high enough to inactivate the endopeptidase. For example, heating the reaction mixture to a temperature of approximately 90° C will substantially heat-inactivate most proteases.

*ii. optional exopeptidase*

The process may further comprise contacting the protein hydrolysate with an exopeptidases. Generally, exopeptidases act only near the ends of a polypeptide chain. Those acting at a free amino terminus liberate a single amino acid residue (i.e., aminopeptidases; APs), a dipeptide (i.e., dipeptidyl-peptidases) or a tripeptide (i.e., tripeptidyl-peptidases). The exopeptidases acting at a free carboxyl terminus liberate a single amino acid (i.e., carboxypeptidases; CPs) or a dipeptide (i.e., peptidyl-dipeptidases). Some exopeptidases are specific for dipeptides (i.e., dipeptidases) or remove terminal residues that are substituted, cyclized or linked by isopeptide bonds. Isopeptide bonds are peptide linkages other than those of a carboxyl group to an $\alpha$-amino group, and this group of enzymes is characterized by omega peptidases.

Typically, the exopeptidase will be a food-grade enzyme having optimal activity at a pH from about 6.0 to about 8.0 and at a temperature from about 50°C to about 60°C. The exopeptidase may be of microbial origin. Examples of exopeptides suitable for use in the process of the invention include aminopeptidase from *Aspergillus oryzae* (SEQ ID NO: 2 in International Application No. WO 96/28542 incorporated by reference in its entirety, aminopeptidase from *Bacillus licheniformis* (UNIPROTE: Q65DH7), carboxypeptidase D from Aspergillus oryzae (UNIPROT: Q2TZ11), carboxypeptidase Y from Aspergillus oryzae (UNIPROT: Q2TYA1), combinations thereof.

The amount of exopeptidase added to the protein hydrolysate can and will vary, depending upon a variety of factors. The amount of exopeptidase may range from about 1 mg to about 5,000 mg of exopeptidase per kilogram of starting protein material. In one embodiment, the amount may range from 1 mg to about 20 mg of exopeptidase per kilogram of starting protein material. In another embodiment, the amount may range from 20 mg to about 1,000 mg of exopeptidase per kilogram of starting protein material. In yet another embodiment, the amount may range from about 1,000 mg to about 5,000 mg of exopeptidase per kilogram of starting protein material.

The pH of the protein hydrolysate may be adjusted to bring the pH of the solution to a level that is optimal for the exopeptidase The pH of the hydrolysate may be adjusted via the addition of either an acid solution or a basic solution, The duration of the exopeptidase reaction can and will vary. Generally speaking, the duration of the hydrolysis reaction may range from a few minutes to many hours, such as, from about 5 minutes to about 5 hours. In a preferred embodiment, the duration of the reaction may be about 30 to 60 minutes. The reaction may be terminated in a manner similar to those discussed above for the endopeptidase reaction.

**c. embodiment**

In an exemplary embodiment, the protein material may be derived from soy, and the soy material may be isolated soy protein. The endopeptidase is GE and the reaction may be conducted at a temperature from about 40°C to about 70°C and at a pH from about 6.0 to about 8.5.

In further exemplary embodiment, the protein material may be derived from soy, and the soy material may be soy protein concentrate. The endopeptidase is GE and the reaction may be conducted at a temperature from about 40°C to about 70°C and at a pH from about 6.0 to about 8.5. The soy protein concentrate may be used in combination with isolated soy protein.

In another exemplary embodiment, the protein material may be derived from soy, and the soy material may be soy flour. The endopeptidase is GE and the reaction may be conducted at a temperature from about 40°C to about 70°C and at a pH from about 6.0 to about 8.5 The soy flour may be used in combination with soy protein concentrate and/or isolated soy protein.

In an exemplary embodiment, the protein material may be derived from soy, and the soy material may be soymilk. The endopeptidase is GE and the reaction may be conducted at a temperature from about 40°C to about 70°C and at a pH from about 6.0 to about 8.5. The soymilk may be used in combination with soy flour, soy protein concentrate, and/or isolated soy protein.

In another exemplary embodiment, the protein material may be derived from soy, and the soy material may be soymilk powder. The endopeptidase is GE and the reaction may be conducted at a temperature from about 40°C to about 70°C and at a pH from about 6.0 to about 8.5. The soymilk powder may be used in combination with soymilk, soy flour, soy protein concentrate, and/or isolated soy protein.

## II. Protein Hydrolysate Composition

Another aspect of the invention encompasses a protein hydrolysate composition. The protein hydrolysate compositions comprises a mixture of polypeptides enriched in a 47 kDa fragment. The polypeptides of the protein hydrolysate composition are between about 10% to about 66% 47 kDa fragments. The polypeptides in the protein hydrolysate composition may be derived from different sources of protein including soy, amaranth, arrowroot, buckwheat, canola; cassava, channa (garbonzo), corn, legume, lentils, lupin, millet, oat, pea, rye sorghum, sunflower, tapioca, triticale, dairy, and combinations thereof. In one embodiment, the protein material may be a soy protein material. A variety of soy protein materials may be used in the process of the invention to generate a soy protein hydrolysate. In general, the soy protein material may be derived from whole soybeans in accordance with methods known in the art. The whole soybeans may be commodity soybeans (i.e., non genetically modified soybeans), genetically modified soybeans, and combinations thereof. Genetically modified soybeans include soybeans with modified protein, oil, or carbohydrate compositions, such as high ß-conglycinin soybeans and high oleic soybeans. Suitable examples of soy protein material include soy extract, soy curd, soy flour, isolated soy protein, soy protein concentrate, soymilk, soymilk powder, and mixtures thereof.

In general, the protein hydrolysates composition, as compared with the starting protein material, will comprise a mixture of polypeptides of varying length and molecular sizes. In an exemplary embodiment, the molecular size of the polypeptides will be enriched in a 47 kDa fragment.

The degree of hydrolysis of the protein hydrolysate composition can and will vary depending upon the source of the protein material and the degree of completion of the reaction.

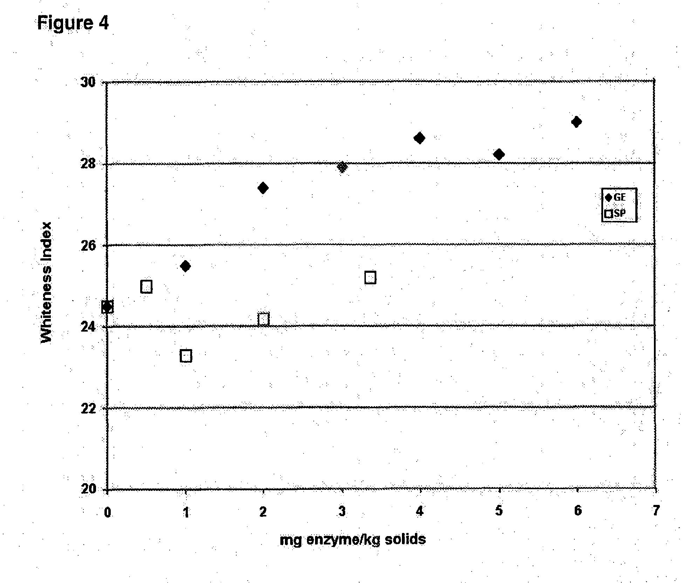

The protein hydrolysate compositions have enhanced whiteness as measured using the whiteness index described herein **(Figure 4)**. At a degree of hydrolysis of 0.5% to 1%, a marked effect is seen on the whiteness of the resulting product when made into either a gel or slurry. The effect of enzyme concentration on whiteness index of the product increases in a dose dependent fashion. Whiter hydrolysates are desirable for use in many food applications such as beverages, gels and meat systems. The soy protein hydrolysates of the present invention are much lighter in color than unhydrolyzed soy protein. The enzyme GE cleaves proteins at C-terminal of glutamic acid and aspartic acid residues. Whiteness is measured using the whiteness index described below.

### The Whiteness Index

The "whiteness index" of a soy protein product refers to the color of the soy-protein-containing composition. Many soy protein-containing compositions will have, to varying degrees, a yellowish or brownish color. In general, the color of these compositions can be "improved," *i.e.,* the "whiteness index" of the product can be increased by the process of the present invention. In general, the whiteness index is determined using a colorimeter which provides the L, a, and b color values for the composition from which the whiteness index may be calculated using a standard expression of the Whiteness Index (WI), WI = L-3b. The L component generally indicates the whiteness or, "lightness", of the sample; L values near 0 indicate a black sample while L values near 100 indicate a white sample. The b value indicates yellow and blue colors present in the sample; positive b values indicate the presence of yellow colors while negative b values indicate the presence of blue colors. The a value, which may be used in other color measurements, indicates red and green colors; positive values indicate the presence of red colors while negative values indicate the presence of green colors. For the b and a values, the absolute value of the measurement increases directly as the intensity of the corresponding color increases. Generally, the colorimeter is standardized using a white standard tile provided with the colorimeter. A sample is then placed into a glass cell which is introduced to the colorimeter. The sample cell is covered with an opaque cover to minimize the possibility of ambient light reaching the detector through the sample and serves as a constant during measurement of the sample. After the reading is taken, the sample cell is emptied and typically refilled as multiple samples of the same material are generally measured and the whiteness index of the material expressed as the average of the measurements. Suitable colorimeters generally include those manufactured by HuhterLab (Reston, VA) including, for example, Model # DP-9000 with Optimal Sensor D 25.

Whiteness index measurements of a 5% by weight solids sample of the suspension before and after treatment are determined using a HunterLab DP-9000 colorimeter including an optical sensor D-25, both manufactured by Hunter Associates Laboratory (HunterLab) (Reston, VA). For the whiteness index measurement in the large scale production platform, protein samples are dispersed on a 5% w/w basis: (5g) is added to deionized water (100 mL). The results obtained using the Hunter Colorimeter are reported in units of L, a, and b. Whiteness Index is calculated from the L and b scale values using the following: Whiteness Index = L - 3b.

**Soy Protein Hydrolysates**

In embodiments in which the protein material is soy, the protein hydrolysate composition may comprise at least one polypeptides selected from the group consisting of SEQ ID NO: 8, SEQ ID NO: 9, SEQ ID NO: 10, SEQ ID NO: 21, SEQ ID NO: 22, SEQ ID NO: 23, SEQ ID NO: 24, SEQ ID NO: 25, SEQ ID NO: 26 and combinations thereof (See Table 6). The invention also encompasses any of the polypeptides that may be purified from the soy protein hydrolysate compositions of the invention. An oligopeptide may be purified by a chromatographic method, such as size exclusion chromatography, ion exchange chromatography, affinity chromatography, fast protein liquid chromatography (FPLC), high performance liquid chromatography (HPLC), and so forth. For example, the polypeptide fragment may be selected from the group consisting of SEQ ID NO; 8, SEQ IQ NO: 9, SEQ ID NO: 10, SEQ ID NO: 21, SEQ ID NO: 22, SEQ ID NO: 23, SEQ ID NO: 24, SEQ ID NO: 25, or SEQ ID NO: 26. Additionally, the invention also encompasses polypeptides that are substantially similar in sequence to SEQ ID NO: 8, SEQ ID NO: 9, SEQ ID NO: 10, SEQ ID NO: 21, SEQ ID NO: 22, SEQ ID NO: 23, SEQ ID NO: 24, SEQ ID NO: 25, or SEQ ID NO: 26. Typically, the oligopeptide may have at least 70, 75, 80, 85, 90, 95, or 99% sequence identity to an oligopeptide having SEQ ID NO: 8, SEQ ID NO: 9, SEQ ID NO: 10, SEQ ID NO: 21, SEQ ID NO: 22, SEQ ID NO: 23, SEQ ID NO: 24, SEQ ID NO: 25, or SEQ ID NO: 26

**Food Products**

A further aspect of the present invention is the provision of a food product comprising any of the protein hydrolysate compositions described herein. Alternatively, the food product may comprise any of the isolated polypeptide fragments described herein.

The selection of a particular protein hydrolysate composition or isolated polypeptide fragment can and will vary depending upon the food product to be formed. Food products of the current invention can include beverages, yogurts, protein bars, whipped food products, cheeses, and other dairy based applications, and extruded food products. It can also be used in meat based gels, and whole muscle meat applications. For food products where it is desirable to minimize the bitterness or astringency sensory attributes, a soy protein hydrolysate composition having a degree of hydrolysis closer to 1% than to 6% is typically selected. Additionally, for food products where it is desirable to minimize the grain and soy/legume sensory attributes, a soy protein hydrolysate composition having a degree of hydrolysis closer to 6% than to 1% is typically selected.

As noted, the protein hydrolysate compositions can be used in a variety of food and/or beverage applications. Non-limiting examples of beverages include smoothies, nutritional drinks, energy drinks, sports drinks, soymilk drinks, flavored soy drinks, rice milk drinks, infant formula, teas, coffee drinks, or combinations thereof.

In another embodiment, the protein hydrolysate can be used in dairy products. The dairy products include, but are not limited to, cheese, ice cream, ice cream products, milkshakes, yogurts, whipping cream, sour cream, cottage cheese, or dairy beverages. Dairy beverages include milk, flavored milk drinks, goat milk, liquid yogurt, smoothies, or buttermilk.

In an alternate embodiment, the protein hydrolysate can be used in a nutritional supplement. The nutritional supplement may be liquid or solid. In another alternate embodiment, the protein hydrolysate can be used in a protein bar, such as a granola bar, a cereal bar, a nutrition bar, or an energy bar.

In still another embodiment, the protein hydrolysate can be used it an extruded food product. Extruded food products include, but are not limited to, cereal-derived products such as breakfast cereals, pasta, breads, baked products (i.e., cakes, pies, rolls, cookies, crackers), puffed snack products, nuggets or tortillas.

In yet another embodiment, the protein hydrolysate can be used in a meat product or a meat analog. Examples of meat products include, but are not limited to, processed meats, comminuted meats, whole muscle meat product, and surimi products. The meat may be animal meat or seafood meat. The meat analog may be a textured vegetable or dairy protein that mimics animal or seafood meat in texture. The meat analog may be part or all of the meat in a food product.

Whipped food products that can be made using these protein hydrolysates include icings and toppings for cakes and tortes, fillings for pastries, pies, and donuts, parfaits and mousses, and meringues.

## DEFINITION

To facilitate understanding of the invention, several terms are defined below.

**Table 3. SQS Lexicon**

| Attribute | Definition | References |
|---|---|---|
| Green | The general category of aromatics associated with green vegetation including stems, grass, leaves and green herbs. | Fresh cut grass, green beans, tomato vines |
| Grain | The aromatics associated with the total grain impact, which may include all types of grain and different stages of heating. May include wheat, whole wheat, oat, rice, graham, etc. | All-purpose flour paste, cream of wheat, whole wheat pasta |
| Soy/Legume | The aromatics associated with legumes/soybeans; may include all types and different stages of heating. | Unsweetened SILK™ soymilk, canned soybeans, tofu |
| Cardboard/ Weedy | The aromatics associated with dried wood and the aromatics associated with slightly oxidized fats and oils, reminiscent of a cardboard box. | Toothpicks, water from cardboard soaked for 1 hour |
| Sweet | The taste on the tongue stimulated by sucrose and other sugars, such as fructose, glucose, etc., and by other sweet substances, such as saccharin, Aspartame, and Acesulfame-K. | Sucrose solutions: 2%, 5%, 10% |
| Sour | The taste on the tongue stimulated by acid, such as citric, malic, phosphoric, etc. | Citric acid solutions: 0.05% 0.08%, 0.15% |
| Salt | The taste on the tongue associated with sodium salts. | Sodium chloride solutions: 0.2%, 0.35%, 0.5% |
| Bitter | The taste on the tongue associated with caffeine and other bitter substances, such as quinine and hop bitters. | Caffeine solutions: 0.05%, 0.08%, 0.15% |
| Astringent | The shrinking or puckering of the tongue surface caused by substances such as tannins or alum. | Alum solutions: 0.005%, 0.007%, 0.01% |

The term "degree of hydrolysis" (DH) refers to the percent of specific peptide bonds that were hydrolyzed (that is, the number of cleaved out of the total number of peptide bonds present in the intact protein). The % DH was estimated using the simplified trinitrobenzene sulfonic acid (S-TNBS) method. This procedure is accurate, reproducible and is a generally applicable procedure for determining the degree of hydrolysis of food protein hydrolysates.

The term "endopeptidase" refers to an enzyme that hydrolyzes internal peptide bonds in oligopeptide or polypeptide chains. The group of endopeptidases comprises enzyme subclasses EC 3.4.21-25 (International Union of Biochemistry and Molecular Biology enzyme classification system).

The term "exopeptidase" refers to an enzyme that hydrolyzes proteins and/or peptides at or near their amino- or carboxyl termini. The group of exopeptidases comprises enzyme subclasses EC 3.4.11-18 (International Union of Biochemistry and Molecular Biology enzyme classification system).

A "food grade enzyme" " is an enzyme that is generally recognized as safe (GRAS) approved and is safe when consumed by an organism, such as a human. Typically, the enzyme and the product from which the enzyme may be derived are produced in accordance with applicable legal and regulatory guidelines.

A "hydrolysate" is a reaction product obtained when a compound is cleaved through the effect of water. Protein hydrolysates occur subsequent to thermal, chemical, or enzymatic degradation. During the reaction, proteins are broken down into polypeptides and free amino acids. These products may be soluble or insoluble in water or water-based buffer solutions.

A "peptide" is a short polymer of amino acids, generally 20 amino acids or less. A "polypeptide" is a polymer of amino acids greater than 20. Both of these polymers contain only primary structure. Polypeptides are formed initially during protein synthesis, and upon "folding" into their native state (i.e., the formation of secondary, tertiary, and quaternary structures) become proteins. In this application, reference to a polypeptide means the generation of a long chain polymer from the hydrolysis of a protein.

A "protein" is a polymer of amino acids that form an active molecule in its native (i.e., undenatured) state. The native state of a protein can have primary, secondary, tertiary, and or quaternary structures. The primary structure of a protein is its amino acid sequence. Proteins typically have secondary structure, which is formed from the interaction of intrachain amino acids. These structures are formed via hydrogen bonding, and are either alpha helixes or "sheets" of interacting amino acids known as beta sheets. Proteins also typically have tertiary structures as well. Tertiary structures are formed through the intrachain interaction of amino acid residues, and occur through ionic, hydrophobic, or other chemical interactions. Some proteins contain one or more "subunits", which interact molecularly to form quaternary structures. Protein subunits are composed of a single polypeptide chain and contain secondary and (usually) tertiary structures.

The term "sensory attribute," such as used to describe terms like "grain," "soy/legume," or "bitter" is determined in accordance with the SQS Scoring System as specifically delineated in Example 11.

The terms "isolated soy protein" or "soy protein isotate," as used herein, refer to a soy material having a protein content of at least about 90% soy protein on a moisture free basis. An isolated soy protein is formed from soybeans by removing the hull and germ of the soybean from the cotyledon, flaking or grinding the cotyledon and removing oil from the flaked or ground cotyledon, separating the soy protein and carbohydrates of the cotyledon from the cotyledon fiber, and subsequently separating the soy protein from the carbohydrates.

The term "soy protein concentrate" as used herein is a soy material having a protein content of from about 65% to less than about 90% soy protein on a moisture-free basis. Soy protein concentrate may also contain soy cotyledon fiber, typically from about 3.5% up to about 20% soy cotyledon fiber by weight on a moisture-free basis. A soy protein concentrate is formed from soybeans by removing the hull and germ of the soybean, flaking or grinding the cotyledon and removing oil from the flaked or ground cotyledon, and separating the soy protein and soy cotyledon fiber from the soluble carbohydrates of the cotyledon.

The term "soy flour" as used herein, refers to full fat soy flour, enzyme-active soy flour, defatted soy flour, partially defatted soy flour, and mixtures thereof. Defatted soy flour refers to a comminuted form of defatted soybean material, preferably containing less than about 1 % oil, formed of particles having a size such that the particles can pass through a No. 100 mesh (U.S. Standard) screen. The soy cake, chips, flakes, meal, or mixture of the materials are comminuted into soy flour using conventional soy grinding processes. Soy flour has a soy protein content of about 49% to about 65% on a moisture free basis. Preferably the soy flour is very finely ground, most preferably so that less than about 1% of the soy flour is retained on a, 300 mesh (U.S. Standard) screen. Full fat soy flour refers to ground whole soybeans containing all of the original oil, usually 18% to 20%. The flour may be enzyme-active or it may be heat-processed or toasted to minimize enzyme activity. Enzyme-active soy flour refers to a full fat soy flour that has been minimally heat-treated in order not to neutralize its natural enzymes.

The term "soymilk" as used herein, refers to an aqueous mixture of any one or more of the following, finely ground soybeans, soy flour, soy flakes, soy concentrate, isolated soy protein, soy whey protein, and aqueous extracts of any one or more of the following: soybeans, soy flakes and soy flour where insoluble material has been removed. Soymilk may comprise additional components including but not limited to fats, carbohydrates, sweeteners, colorants, stabilizers, thickeners, flavorings, acids, and bases.

The term "soymilk powder" as used herein, refers to a dewatered soymilk. Soymilk may be dewatered by many processes that include but are not limited to spray drying, tray drying, tunnel drying, and freeze drying.

The term "simplified trinitrobenzene sulfonic acid (S-TNBS) method" as used herein, refers to an accurate, reproducible and generally applicable procedure for determining the degree of hydrolysis of food protein hydrolysates. For this, 0.1 g of the soy protein hydrolysate was dissolved in 100 mL of 0.025 N NaOH. An aliquot (2.0 mL) of the hydrolysate solution was mixed with 8 mL of 0.05 M sodium borate buffer (pH 9.5). Two mL of the buffered hydrolysate solution was treated with 0.20 mL of 10% trinitrobenzene sulfonic acid, followed by incubation in the dark for 15 minutes at room temperature. The reaction was quenched by adding 4 mL of a 0.1 M sodium sulfite-0.1 M sodium phosphate solution (1:99 ratio), and the absorbance was read at 420 nm. A 0.1 mM glycine solution was used as the standard. The following calculation was used to determine the percent recovery for the glycine standard solution: [(absorbance of glycine at 420 nm - absorbance of blank at 420 nm) x (100/0.710)]. Values of 94% or higher were considered acceptable. (Jens Adler-Nissen (1979) "Determination of the Degree of Hydrolysis of Food Protein Hydrolysates by Trinitrobenzenesulfonic Acid," J. Agric Food Chem. 1 27(6): 1256-1262.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above compounds, products and methods without departing from the scope of the invention, it is intended that all matter contained in the above description and in the examples given below, shall be interpreted as illustrative and not in a limiting sense.

## EXAMPLES

The following examples illustrate various embodiments of the invention.

### Example 1. Preparation of a Soy Protein Product From Defatted Soy Flour

About 30 lbs of defatted soy flour was added to 250 lbs of water to make a 10% solids slurry at pH 6.5. The slurry was heated to 54°C (130°F) and stirred for 30 minutes without pH adjustment. The pH of the slurry was then adjusted to pH 7.0 using 80 ml NaOH (0.6% on solid basis), forming a neutral slurry. The neutral slurry was heated to 160.5°C (321°F) and held for 27 seconds. The hot neutralized slurry was cooled down to 53°C (128°F) and then dried in a spray dryer to form a soy protein product. Solids to the dryer was 9.9% at pH 6.84.

### Example 2. Preparation of a Soy Protein Product From Defatted Soy Flour Treated With 2.4 mg GE

About 30 lbs of defatted soy flour was added to 250 lbs of to make a 10% solids slurry at pH 6.5. The slurry was heated to 54°C (130°F) and 2.4 mg GE enzyme protein/kg protein solid was added and held for 30 minutes with agitation. After 30 minutes the pH was adjusted to 7.0 using 70 ml NaOH (0.6% on solid basis) to form an enzyme-treated neutral slurry. The pH had dropped to 6.23 during the enzyme treatment. The enzyme-treated neutral slurry was heated to 160.5°C (321°F) and held for 27 seconds. The hot neutral enzyme-treated slurry was cooled to 53°C (128°F) and then dried in a spray drier. Solids to the dryer was 9.6% at pH 6.82.

### Example 3. Preparation of a Soy Protein Product From Defatted Soy Flour Treated With 4.8 mg GE

30 lbs of defatted soy flour was added to 250 lbs of water to make a 10% solids slurry at pH 6.5. The slurry was heated to 54°C (130°F) and 4.8 mg GE enzyme protein/kg protein solid was added and held for 30 minutes with agitation. After 30 minutes the pH was adjusted to 7.0 using 100 ml NaOH (0.8% on solid basis) to form an enzyme-treated neutral slurry. The pH had dropped to 6.32 during the enzyme treatment. The enzyme-treated neutral slurry was heated to 160.5°C (321°F) and held for 27 seconds. The hot neutral enzyme-treated slurry was cooled to 53°C (128°F) and then dried in a spray driver. Solids to the dryer was 9.6% at pH 6.84.

The protein compositions of Examples 1 to 3 were analyzed by SDS-PAGE using standard procedure, and Figure 2 presents an image of the gel. This analysis revealed that GE selectively cleaved the extension region of a and α' subunits of ß-conglycinin thereby releasing it from the hydrophobic core region, a 47 kDa fragment.

### Example 4. Enrichment of 47 kDa Polypeptides by Salt Treatment

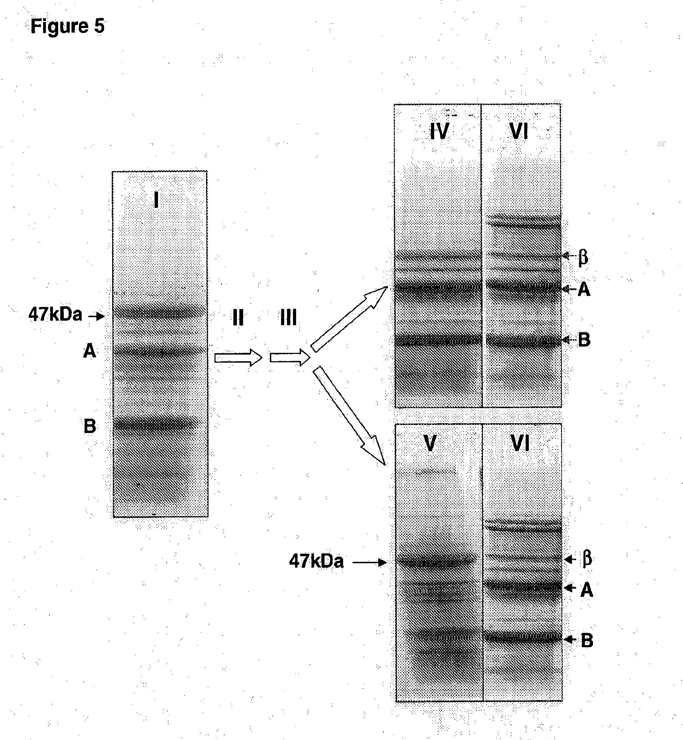

The soy protein product from Example 2 was further treated by 2% salt (NaCl). The procedure was as follows: a 2% salt solution was prepared by dissolving 2g of NaCl in 100 ml of water. 2g of each of the soy protein products produced in Example 2 were combined with 50g of the salt solution and mixed to obtain dispersions at 75°C for 30 minutes. The dispersion was centrifuged at 3000 x G for 10 minutes to separate the precipitate from the supernatant. The supernatant is identified as containing the glycinin (11S) and subunit of ß-conglycinin (7S), and the precipitate is identified as containing the 47 kDa polypeptide as presented in **Figure 5.**

### Example 5. Enrichment of 47kDa polypeptide by anionic chromatography

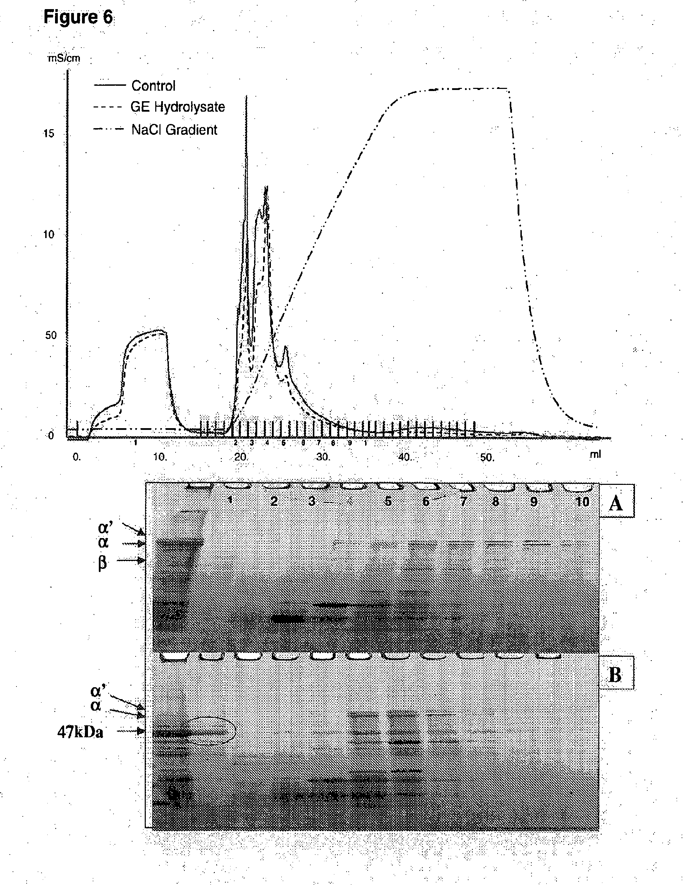

An anionic exchange column, Q Sepharose XL (GE Healthcare) was used to separate the 47 kDa polypetides from other protein components in GE treated protein. One milliliter of 1% protein solution was passed through the 1-ml column at 1 ml/min flow rate then another ml of deionized water was washed through the column. The water eluted samples were identified as mainly 47kDa polypeptides as shown in Figure 6, fraction 1 in the chromatograph of GE hydrolysate and sample 1 in SDS-PAGE gel B. The result indicates that the 47kDa polypeptide is either very hydrophobic or cationic.

### Example 6. Water Binding Ability measured by Thermo Hydration Ratio

The Thermo Hydration Ratio (THR) test, a test to determine the hydration ratio for soy protein in a 2% salt solution with heat. The THR values indicate the water binding ability of soy protein in various applications, e.g. meat, yogurt, pudding. In the THR test procedure, a 2% salt solution is prepared and 50 grams of this solution is combined with 2 grams of a soy protein product, on a moisture free basis and mixed to obtain a dispersion. The dispersion is centrifuged to separate solids from supernatant. The supernatant is filtered through a 325 mesh stainless steel fabric to obtain a filtrate. The filtrate is then weighed. The THR is determined with the below formula:

$$THR = \frac{A + B - C}{D}$$

where

A is the weight of salt solution. B is the sample weight of the soy protein product, adjusted to include moisture. If the moisture content of a soy protein product is 4.22%, the moisture free content is 95.78%. Then 2 grams of a soy protein product on a moisture free basis weighs 2.09 grams when moisture is included (2/.9578 = 2.09).
C is the weight of filtrate.
D is a 2 gram weight of the soy protein product on a moisture free basis.

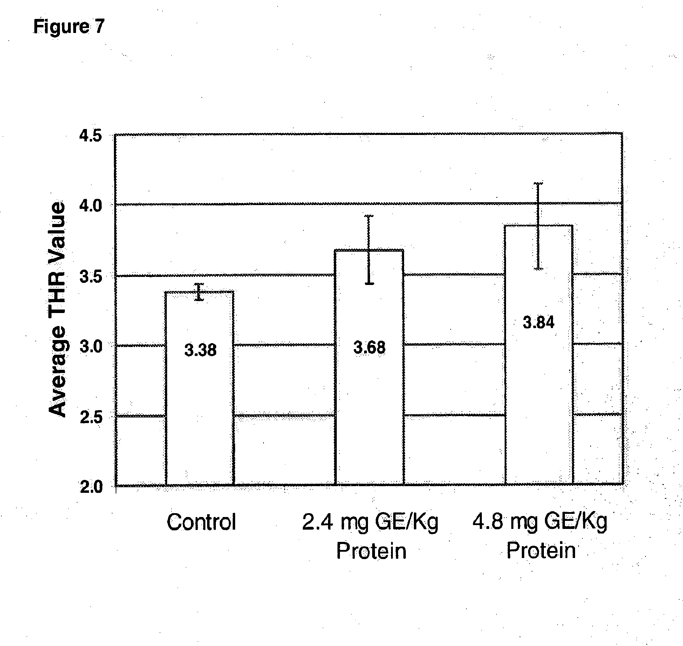

The THR test results of GE modified proteins and control are shown in Figure 7. The GE hydrolyzed proteins have significantly greater THR than the control. This indicates that GE modified protein can hold more water in the presence of salt. This result implies that the GE modified protein may have the potential in meat, yogurt and pudding applications.

**Example 7. Rapid Visco Analyzer (RVA) analysis**

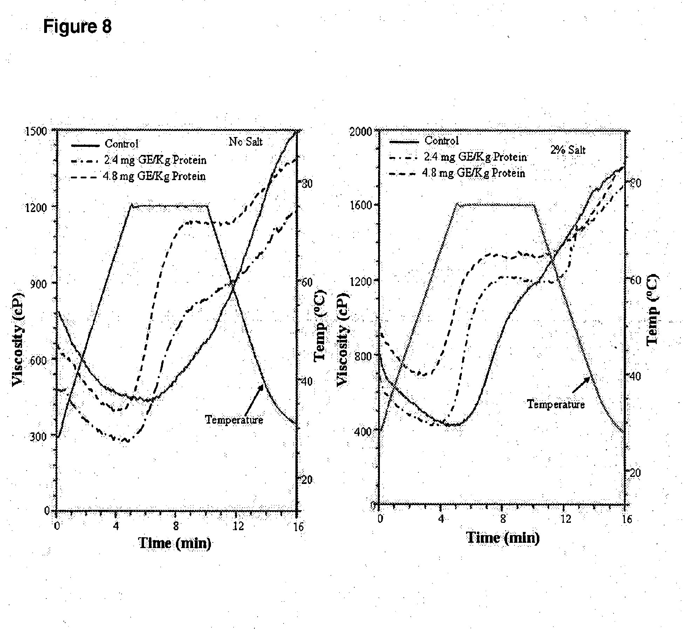

The Rapid Visco Analyzer (RVA) is a heating and cooling viscometer that can provide a viscosity method with precise control of temperature and shear over controlled times.
Soy protein concentrate and flours contain mainly protein and fiber which are sensitive to the heat treatment. The RVA viscosity profile corresponding to the heating process may reflect the heat sensitivity of soy protein products. Thus comparing the RVA heating profiles between samples can be a useful tool to predict their heat responses of soy protein products in some food applications.
The program is designed to record the viscosity of a 10% protein slurry during the heating course from 25°C to 75°C (hold for 5 minutes) then cooled to 25°C.
As shown in **Figure 8,** GE hydrolysis changed the heat sensitivity of soy protein, the higher dose of GE treatment the more significant change in viscosity. The result indicates that the GE modified soy protein may thicken or gel at low temperature in food applications such as cheese, yogurts, pudding, and so on.

**Example 8. Bench Top Hydrolysis of Soy Protein Isolate Using GE Versus Hydrolysis of the Acid Precipitated Soy Protein Obtained from Soy Flakes**

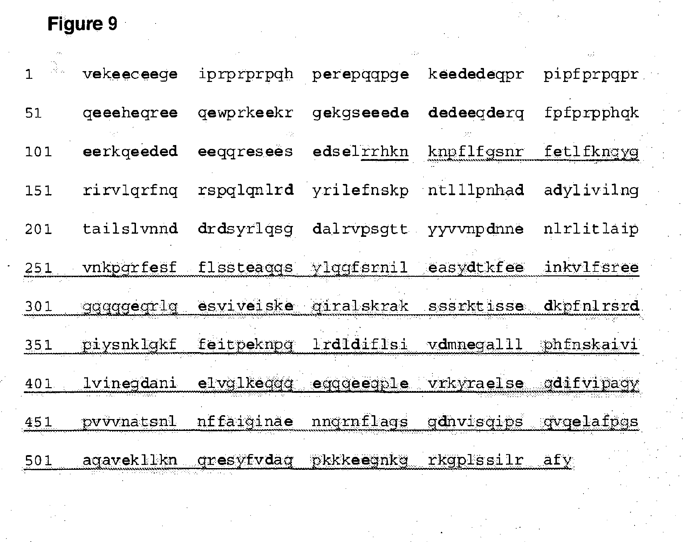

Large numbers of potential cleavage sites in the extension region, shown in Figure 9 makes this region susceptible to GE hydrolysis When the protein is in native state the extension region is hydrolyzed preferentially where as in more denatured state hydrolysis takes place throughout the molecule.
Soy protein isolate made using standard isolate procedure known in the art, which involve protein extraction, isoelectric precipitation, reslurrying the protein, UHT pasteurization followed by spray drying was used as a substrate. Soy protein isolate slurry with 10% solid content was made by mixing 10 grams of soy protein isolate with 90 grams of water. The pH was adjusted to 8.0 using 0.1 N NaOH, enzyme hydrolysis with GE was carried out at 50°C for 30 minutes enzyme concentration of 12, 24, and 48 mg of enzyme protein/ 1 kg of soy protein isolate on a dry matter basis were used, hydrolysis was terminated by heating the slurry at 85°C for 5 minutes. Samples were cooled and subjected to SDS-PAGE analysis, the results are shown in **Figure 1.**
The hydrolysis procedure disclosed above was followed using an acid precipitated soy protein curd slurry as the substrate. The slurry was obtained from a standard isolate procedure, which involved protein extraction, isoelectric precipitation, reslurrying of the protein, UHT pasteurization followed by spray drying. Slurry samples were collected from the reslurrying step before UHT pasteurization. The slurry was at 10% solid, the pH was adjusted to 8.0, using 0.1 N NaOH, enzyme hydrolysis with GE was carried out at 50°C. The hydrolysis was carried out for 30, 60, and 120 minutes. Enzyme concentration of 24, 48, and 96 mg of enzyme protein/1 kg of soy protein isolate on a dry matter basis were used. Hydrolysis was terminated by heating the slurry of 85°C for 5 minutes. Samples were cooled and subjected to SDS-PAGE analysis, the results are shown in **Figure 3.** When **Figure 1** is compared to **Figure 3** it is evident that hydrolysis patterns differ greatly based on the state of the substrate, i.e. denatured versus native. It can also be noted that in native state, even at high enzyme concentrations and over extended period of hydrolysis, 47 kDa fraction was relatively stable to hydrolysis, making it possible to harvest this fraction with a robust manufacturing process.

**Example 9. Preparation of 47kDa Enriched Fraction From Soy Bean Flour:**

500 lbs of defatted soy flour was mixed with 5000 lbs of water to make a 10% solids slurry at 32°C. The slurry was separated by centrifugation and first supernatant and first precipitate were collected. The first precipitate was re-slurried with 3000 lbs of water and the slurry separated by centrifugation. The second precipitate was discarded and the second supernatant collected and added to the first supernatant to form a combined supernatant. The pH of the combined supernatant was adjusted to 4.53 with HCl to form a curd. The curd was separated at 32 °C by centrifugation and reslurried with water to 10% solid.

One-third of the curd slurry was removed and pH adjusted to 7.2 using a NaOH/KOH blend to form a neutral curd. The neutral curd was pasteurized by heating to 157°C for 9 seconds and cooled to 61°C. The cooled neutral curd was spray dried to obtain control soy protein isolate.

Two-thirds of the curd slurry was removed and pH adjusted to 8.0 using NaOH/KOH blend. The pH 8.0 curd slurry was heated to 55 °C and dosed with GE.

The enzyme containing slurry was held for 30 minutes with agitation to complete the enzyme treatment. The enzyme treated slurry was then divided into two portions.

The first enzyme treated portion was pH adjusted to 7.2 with NaOH/KOH blend, heated to 161 °C, held for 9 seconds, cooled to 55 °C and spray dried to form an enzyme treated soy protein isolate.

The second enzyme treated portion was pH adjusted to 4.5 with HCl to form an enzyme treated curd and then separated by centrifugation at 60°C. The enzyme treated curd was slurried in water to 10% solids content, neutralized to pH 7.2 with NaOH/KOH blend, heated to 160°C for 9 seconds, cooled to 56 °C, and spray dried for form an enzyme treated soy protein isolate.

There were two enzyme treatments: the first received a dose of 6 mg GE per kg of solids, and the second received a dose of 12 mg GE enzyme protein per kg of solids.

**Figure 10** shows the SDS-PAGE of the hydrolysates generated in this example.

**Example 10. Use of Novel Enzyme GE to Create Whiter Soy Protein Hydrolysates**

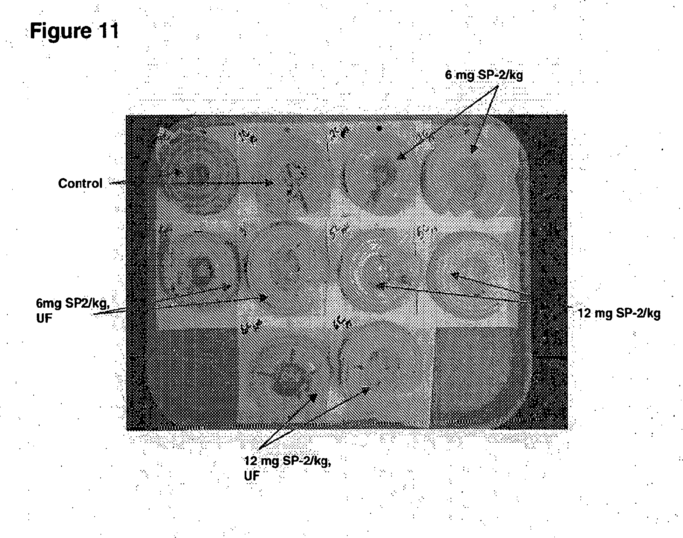

Protein products produced in Example 9 were used to make gels. To make the gels, samples were dispersed with water at 1:6 ratio, mixed and put in 6oz. cans and cooked in a boiling water bath for 30 minutes to form the gels. The pictures of these gels are shown in **Figure 11.** It is evident from **Figure 11** that the GE treated products yielded much whiter gels.

Whiteness index of slurries made from protein products produced in Example 9 were measured at 5% concentration and is shown in Table 4.

**Table 4. Whiteness Index of GE treated samples, Hunter measurements**

| Sample | Whiteness Index |
|---|---|
| Control | 23.2 |
| Sample with 6mg GE/kg slurry solids | 29.5 |
| Sample with 12mg GE/kg slurry solids | 35.4 |

The whiteness index for the protein product treated with the higher GE concentration was 12.2 units higher than GE untreated protein product

**Example 11. Sensory Analysis of the GE Hydrolysates**

A proprietary sensory screening method, the Solae Qualitative Screening (SQS) method, was used to assess the flavor characteristics of the GE hydrolysates prepared in Example 9. This method is based upon a direct comparison between a test sample and a control sample, and it provides both qualitative and directional quantitative differences. A panel often assessors was provided with aliquots of each sample (diluted to a 5% slurry) and a control sample that was 5% slurry of untreated isolated soy protein.

The evaluation protocol comprised swirling a cup three times, while keeping the bottom of the cup on the table. After the sample sat for 2 seconds, each taster sipped about 10 mL (2 tsp), swished it about her/his mouth for 10 seconds, and then expectorated. The taster then rated the differences between the test sample and the control sample according to the scale presented in Table 5.

**Table 5. SQS Scoring System**

| SQS Score | Scale | Definition |
|---|---|---|
| 5 | Match | The test sample has virtually identical sensory characteristics to the control sample by appearance, aroma, flavor and texture. |
| 4 | Slight difference | The test sample has one or multiple 'light' differences from the control sample. These differences might not be noticed if not in a side-by-side comparison with the control. |
| 3 | Moderate difference | The test sample has one or multiple 'moderate' differences from the control sample. These differences would be noticeable in a side-by-side comparison of the two samples after one tasting of each. |
| 2 | Extreme difference | The test sample has one or multiple 'extreme' differences from the control sample. These differences would be noticed even if not in a side-by-side comparison. |
| 1 | Reject | The test sample has obvious defects that make it different from the control sample. |

If a test sample was rated as different from the reference (i.e., had an SQS score of 2, 3, or 4), then the test sample was further evaluated to provide diagnostic information on how the test sample differed from the control sample. Thus, if the test sample had slightly more, moderately more, or extremely more of an attribute (see Table 5) than the control sample, then scores of +1, +2, +3, respectively, were assigned. Likewise, if the test sample had slightly less, moderately less, or extremely less of the attribute than the control sample, then scores of -1, -2, -3, respectively, were assigned. This analysis provided an assessment of the directional quantitative differences between the test sample and the control sample.

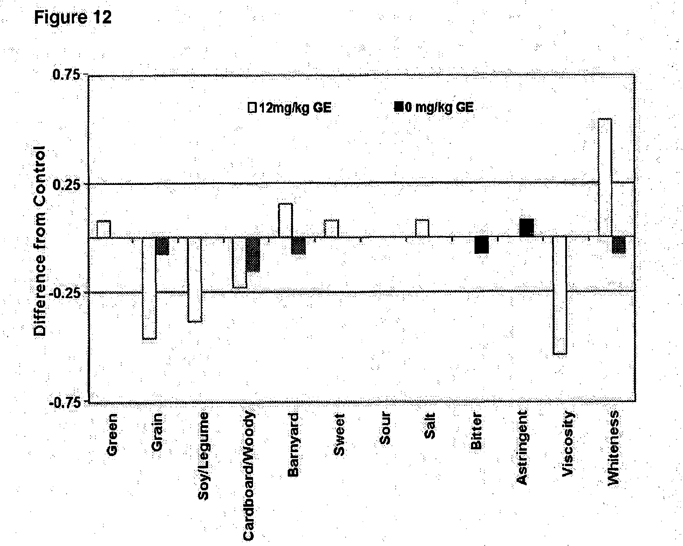

The diagnostic scores (differences from the reference) for each GE hydrolysates are presented in **Figure 12.** The GE hydrolysates had slightly less grain, and soy/legume flavor characteristics relative to the control (untreated isolated soy protein).

**Example 12: Whiteness Index of Bench Top Hydrolysis Of Soy Protein Isolate Using GE versus SP Enzyme.**

a) **GE hydrolysis:** Samples were generated as described in Example 8

b) **SP hydrolysis:** Same protocol described in Example 8 was followed for SP hydrolysis, except the enzyme dose of SP. Enzyme dose used for SP was 3.3 mg enzyme/1kg of isolate and 6.6 mg enzyme/1kg of isolate. Hydrolysis was terminated by heating the slurry at 85°C for 5 minutes. Samples were cooled and analyzed.

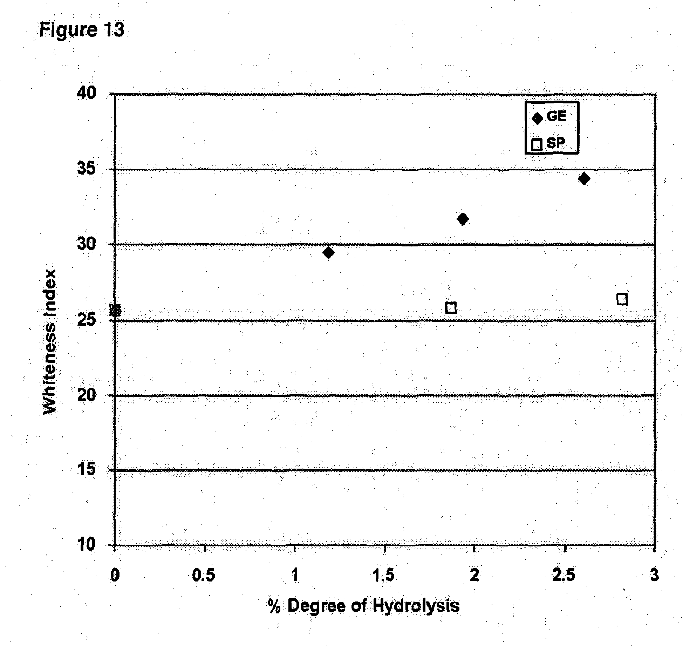

Whiteness index for the hydrolysate slurry was measured for samples prepared with both GE and SP enzyme and they are shown in **Figure 13. %** DH for each hydrolysate were obtained by using S-TNBS methodology.

While the whiteness index for commodity hydrolyzed samples increased with %DH when hydrolyzed with GE, SP led hydrolysis in the same %DH range had no effect on the whiteness index.

**Example 13: Bench top hydrolysis of acid curd prepared in pilot plant using smaller doses of GE and SP enzyme.**

Commodity acid curd made in the pilot plant as described in Example 9 was used. Solid content of the curd was 10%, the pH was adjusted to 8.0 and the sample was aliquoted in several 200g batches. Enzyme hydrolysis with GE was carried out at 50°C for 30 minutes at 0, 1, 2, 3, 4, 5, and 6 mg GE/kg solids of the curd on dry matter basis, using curd batch size of 200 g. Enzyme hydrolysis with SP was carried out at 50°C for 30 minutes at 0, 0.5, 1, 2 and 3.36 mg SP/kg solids of the curd on dry matter basis, using curd batch size of 200g. Hydrolysis was terminated by heating the slurry at 85°C for 5 minutes. Samples were cooled and tested for whiteness index. Though the dosages of enzyme used was really shall, a dose dependent increase was seen in whiteness index in GE treated samples, such effect was not seen for SP treated samples **(Figure 4).**

**Example 14. Hydrolysis of Soy Protein with GE**

_Hydrolysis Reactions._ The starting material was 10% soy protein isolate (e.g., SUPRO® 500E) suspended in 0.1 M sodium phosphate, pH 8.5. The pH of the protein slurry was adjusted to 8.0 with HCl. The protein slurry was heated to about 70°C and the mixture was hydrolyzed with serine protease (GE) from _Bacillus licheniformis._ GE was used at a concentration of 19.5 mg, 39.0 mg, 78.1 mg, 156.3 mg, or 312.5 mg of protease enzyme protein per kg of soy protein isolate. The hydrolysis reaction was allowed to proceed at 70°C for 120 minutes. The reaction was stopped with the addition of 1 M sodium formate, pH 3.7, such that the pH of the mixture was about 4.0 and the final concentration of soy protein in the hydrolysate was 5%.

**Example 15. Identification of Peptides in GE Hydrolysates**

Peptide fragments in the GE hydrolysates prepared in Example 14 were identified by liquid chromatography mass spectrometry (LC-MS). Table 6 presents peptide fragments derived from glycinin subunit G2 and chain A of proglycinin.

**Table 6.**

| Protein | 60 minutes | | 120 minutes | |
|---|---|---|---|---|
| | SEQ ID NO | Sequence | SEQ ID NO | Sequence |
| α-subtinit of β-conglycinin | 2 | NKVLFSREE | 2 | NKVLFSREE |
| | 3 | INKVLFSREE | 3 | INKVLFSREE |
| | 4 | ASYDTKFEE | 17 | LVGLKE |
| | 5 | KLLKNQRE | 18 | NFLAG |
| | 6 | LLLPN | 19 | QPRQE |
| | 7 | FKNQ | 20 | SVIVE |
| | | | 6 | LLLPN |
| | | | | |
| ß-subunit of β-conglycinin | 8 | LKVREDE | 21 | FVDAQPQQKEE |
| | 9 | GVIVE | 22 | SYFVDAQPQ QKEEE |
| | 10 | RIVQ | 23 | KDNVVRQIE |
| | | | 24 | LVGIKE |
| | | | 25 | QPQCQKE |
| | | | 26 | RLLKK |
| Glycinin Gy 4 A5A4B3 | 11 | KLQSPDDE | 27 | RKQIVTVE |
| | 12 | NSQHPE | 28 | QQEEEEE |
| Glycinin subunit G1 (proglycinin A1aB1b) | 13 | FSSREQPQQ NE | | |
| | 14 | FLKYQQE | | |
| p24 oleosin isoform A | 15 | AGVKAPSIYH SE | 29 | VGQDIQSKA QDTRE |
| | 16 | NLAAAAKHHL AE | 30 | AAAAKHHLA E |
| lox3 | | | 31 | GLTVDE |
| | | | 32 | FLWG |
| glycinin | | | 33 | ITSSKFNE |

(continued)

| Protein | 60 minutes | | 120 minutes | |
|---|---|---|---|---|
| | SEQ ID NO | Sequence | SEQ ID NO | Sequence |
| A3B4 | | | 34 | KFNE |
| 2S albumin precursor | | | 35 | HIMEK |
| | | | 36 | KMEK |
| allergen Bd 28K | | | 37 | KMEE |
| | | | 38 | KFLQ |

**Experimental Example 1. Acid Beverage Containing Soy Protein Hydrolysates.**

An acid beverage according to the present invention is prepared. 130.0 parts soy protein ingredient and 8,539 parts deionized water are added to a vessel. The contents are mixed under high shear until evenly dispersed. The dispersion is then heated to between about 74°C to about 79°C (about 165°F to about 175 °F) and mixed for an additional 10 minutes. Then added with mixing are 1, 180 parts high fructose corn syrup, 131 parts apple juice concentrate (68 Brix) and 20.0 parts anhydrous citric acid. The pH is adjusted to 3.8-4.0 with 85% citric acid. The contents are homogenized at 2500 pounds per square inch in the first stage and at 500 pounds per square inch in the second stage followed by pasteurization at 107°C for 7 seconds. Bottles are hot filled with the beverage and then placed in an ice bath to bring the temperature of the beverage to about room temperature and placed in the refrigerator.

**Experimental Example 2. A Dry Blend Containing the Soy Protein Hydrolysates.**

The following Experimental Example illustrates the preparation of the dry, blend containing the protein of this invention with components listed in Table 7 below.

**Table 7**

| Component | Parts by Weight | Grams per Serving |
|---|---|---|
| Soy Protein Hydrolysates | 56.85 | 16.89 |
| Fructose | 20.23 | 6.01 |
| Sucrose | 20.22 | 6.01 |
| Dry Cream Extract | 1.01 | 0.30 |
| Ice Cream Vanilla Flavor | 1.35 | 0.40 |
| Sodium Chloride | 0.34 | 0.10 |
| Total | 100.00 | 29.71 |
| Ingredients are added to a vessel and mixed to form a dry blend. | | |

**Experimental Example 3 -A beverage containing the dry blend as prepared in Experimental Example 2**

A ready to drink beverage is prepared by adding a dry blend as prepared in Experimental Example 2 to a liquid. Order of addition of is of no importance.

Within the ready to drink beverage, the liquid is present at from about 85% up to about 95% by weight of the total composition, and the pH of the ready to drink beverage is from about 6.8 up to about 7.4.

Experimental Example 3 is the inventive ready to drink beverage prepared by adding 29.71 grams of the product of Experimental Example 2 to 240 ml of skim milk. The contents are blended for 30 seconds.

**Experimental Example 4 - A beverage containing the dry blend as prepared in Experimental Example 2**

A ready to drink beverage is prepared by adding a dry blend as prepared in Experimental Example 2 to a liquid. Order of addition of is of no importance.

Within the ready to drink beverage, the liquid is present at from about 85% up to about 95% by weight of the total composition, and the pH of the ready to drink beverage is from about 6.8 up to about 7.4.

Experimental Example 4 is the inventive ready to drink beverage prepared by adding 29.71 grams of the product of Experimental Example 2 to 240 ml of water. The contents are blended for 30 seconds.

**Experimental Example 5 Unflavored Low Fat Soymilk Containing the Soy Protein Hydrolysates**

Serving size: 8.5g protein/250g.

**Table 8 Formula for Unflavored Low Fat Soymilk**

| Ingredients | % in formula |
|---|---|
| Distillled Water | 89.4 |
| Potassium citrate | 0.25 |
| Soy protein hydrolysate* | 3.8-4.2 |
| Maltodextrin | 4-4.4 |
| Sugar | 1.4 |
| High Oleic Sunflower oil | 0.72 |
| Carrageenan | 0.03 |
| * Percentage of soy protein hydrolysate used in formula was adjusted depending on protein content as is ||

Disperse citrate in 60°C (140°F) water. Increase mixing speed and disperse protein into water. After protein is thoroughly dispersed increase slurry temperature to 75°C (167°F), reduce mixing speed and continue mixing 10 minutes. Preblend maltodextrin, sugar and carrageenan, add to protein slurry and continue mixing at low speed for 5min. Add sunflower oil to slurry and continue mixing at slow speed until homogenous for approximately 3 minutes. Adjust slurry pH using 45% potassium hydroxide to between 7.0 and 7.2. Process as follows: homogenization, pasteurization and cooling. Heat product to 72°C (162°F) and homogenize at 750psi, second stage; 2250 psi, first stage. Pre-heat slurry to 100°C (212°F) and UHT at 141 °C (286°F) for 6 seconds. Cool product to 31 °C (88°F) and package in sterilized bottles. Store refrigerated.

**Experimental Example 6 Unflavored Beverage Containing 50% Soy protein Hydrolysates and 50% Skim Milk.**

Serving size: 8g protein/260g

**Table 9 Unflavored Beverage formula**

| Ingredients | % in formula |
|---|---|
| Distilled Water | 43.9 |
| Soy protein hydrolysates* | 1.71-1.9 |
| Skim milk | 50 |
| Maltodextrin | 1.95-2.12 |
| Sugar | 1.0 |
| High Oleic Sunflower oil | 0.84 |
| Sodium citrate, dehydrate | 0.05 |
| Magnesium phosphate, dibasic | 0.038 |
| Cellulose gel | 0.25 |
| Carrageenan | 0.02 |
| Vitamin/mineral premix | 0.006 |
| * Percentage of soy protein hydrolysates used in formula was adjusted depending on protein content as is ||

Disperse citrate in all deionized water at 15°C (59°F) using moderate speed mixing. Disperse SUPRO® Plus in water. After all lumps are dispersed heat slurry to 77°C (170°F) and continue mixing at slow speed for 10 minutes. Dry blend maltodextrin, sugar, vit/min premix, magnesium phosphate, cellulose and carrageenan. Add dry blend to protein slurry and continue mixing 5 minutes. Add sunflower oil and continue mixing 3 minutes. Measure slurry pH and adjust pH if necessary to pH 6.9 to 7.1 using either 50% citric acid solution or 1N NaOH. Heat skim milk slowly to 72°C (162°F) and add protein slurry to heated skim milk. Add flavoring agents and mix until completely incorporated into slurry. Blend for 3 minutes with slow mixing and record final slurry pH. Process as follows: Heat product to 72°C (162°F) and homogenize at 750 psi, second stage, 2250 psi, first stage. Pre-heat slurry to 104°C (220°F) and UHT at 141 °C (286°F) for 6 seconds. Cool product to 31°C (88°F) and package. Store refrigerated.

**Experimental Example 7 Vanilla Flavored Weight Management Beverage Containing Calcium Caseinate, Non-Fat Dry Milk (NFDM) and Soy Protein Hydrolysates as the Protein Sources**

Serving size: 10g protein/11 oz

**Table 10 Vanilla Flavored Formula**

| Ingredients | % in formula | |
|---|---|---|
| | All milk protein | Soy containing 20% Ca caseinate replacement |
| Distilled Water | 82.4 | 82.5 |
| Soy protein hydrolysate | 0.0000 | 0.68-0.71 |
| NFDM | 6.39 | 6.39 |
| Sucrose | 7.0 | 7.0 |
| Gum Arabic | 1.31 | 1.31 |
| Calcium Caseinate (85.5%) | 0.68 | 0 |
| Cellulose gel | 0.35 | 0.35 |
| Canola Oil | 0.77 | 0.74 |
| Potassium Citrate | 0.14 | 0.13 |
| Sodium Citrate | 0.05 | 0.04 |
| Lecithin | 0.07 | 0.07 |
| Carrageenan | 0.04 | 0.104 |
| Calcium Carbonate | 0.06 | 0.03 |
| Magesium Carbonate | 0 | 0.02 |
| Magnesium Phosphate, dibasic | 0.25 | 0.21 |
| Vit/Min premix | 0.07 | 0.07 |
| Vanilla flavor | 0.40 | 0.40 |

Prepare a pre-blend as follows: The carrageenan and cellulose with a small portion of the formula sucrose. The gum arabic and remaining sucrose. Mix canola oil.

Then blend: Add the potassium and sodium citrate to water using high shear mixing. Then disperse the preblended carrageenan and cellulose. Mix for 5 minutes. Disperse the soy protein, calcium caseinate and NFDM and begin heating to 65°C (150°F). Hydrate for 15 minutes, reduce mixing speed after reaching 65°C (150°F). Heat canola oil/emulsifier blend to about 70°C (158°F) to dissolve emulsifiers. this may require some mixing. Then add hot oil mix to batch tank, blend 5 minutes, foam will disperse. Add sucrose/gum arabic blend, calcium carbonate, magnesium phosphate, magnesium carbonate and vitamin premix, mix for 10 minutes. Add vanilla flavor and adjust pH with 45% KOH to 7.0-7.2. UHT 286F/6sec, 2500/500 psi

**Experimental Example 8 Dry Blended Beverage Performance Beverage Model Containing Soy Protein Hydrolysates**.

## Table 11 Performance Beverage Formula

| Ingredients | % in formula |
|---|---|
| Soy protein hydrolysates | 16.2-16.9 |
| Whey Protein Isolate (WPI) (92.7% protein as is) | 15.81 |
| Sugar | 3.25 |
| Fructose | 3.25 |
| Cocoa | 3.00 |
| Fat Powder | 1.40 |
| Xanthan Gum | 0.40 |
| Vitamin Premix, | 0.06 |
| Sucralose | 0.04 |
| Sweetness Enhancer, | 0.30 |
| Chocolate flavor | 0.65 |
| Cream | 0.20 |
| Total | 44.8-45.3 |

Clean and sanitize mixer. Sieve soy protein and cocoa powder. Mix all ingredients for 15 minutes at medium speed. Store dry powder in sanitized containers.

**Experimental Example 9 Extrudates Containing Soy Protein Hydrolysates.**

The process starts with the formulation; the blend of ingredients to obtain certain level of protein; example: 10% - 90% protein dry basis. To achieve the proper % of protein, the protein can be a combination of different sources for protein such as: ISP, soy protein concentrate, soy flour, other vegetable protein, dairy proteins, etc. The soy protein hydrolysates of the current invention are also used as part of the protein component. For the other portion in the formulation to meet 100%, the other components can include moisture, ash, fat, carbohydrates and other ingredients known to those skilled in the art. Multiple sources for these ingredients can be used, such as starches, cereal grains flours or derivatives from cereal grains, fibers, gums, syrups, fat, salts, sugars, flavors, etc.

After defining the composition of the final product, usually dry blending is used to homogenize the dry ingredients, which are sent to the feeding extrusion hopper. The formulation is then fed into a preconditioner, if used, or directly into the extruder. Water and/or steam are injected into preconditioner to pre-hydrate and/or preheat the blend. Water will be injected into preconditioner at about 0 % - 30 % based in dry feed and steam from 0 % - 15 %. Rate of materials (dry, liquid and steam) from preconditioner can be from 0.2 - 150 (Lb/ Min) or greater depending on the extruder's barrel diameter and screw channel open area, the diameter of the barrel and the openness of the screw deal with feed rate.

The ability of the extruder to appropriately "cook" the dry or preconditioned feed will depend on the L/D, length to diameter ratio. Typically for high protein products, greater than 20% total protein dry basis, an extruder with an L/D greater than 15:1 is used. Ingredients are fed into the extruder running at typical screw speed of 200 rpm - 1000 rpm. Additional water, steam, syrups and/or other ingredients are incorporated into the extruder barrel; typical ranges (0% - 40%) to achieve the proper product characteristics in the different areas of extrusion application:

Direct expansion "breakfast cereals, nuggets, snacks, etc.".
Texturized products. Texturized vegetable protein (TVP), etc".
High moisture extrusion greater than 50% moisture "Meat type texture, pasta, etc."

These ingredients may be incorporated into the extruder in different points of the barrel and they are sheared by a predesigned screw configuration to achieve the proper final product attributes. A die is used at the end of the extruder to create back pressure and extrusion filling to create the proper cooking, extrudate temperature and pressure to obtain the right expansion when the material exits the die. The die must be symmetric for best results and can have any geometry or shape available.

The Extruder produces a continuous rope which can be reduced to small pieces using secondary devices such as face cutting, external cutting, and other devices. A face cutter is used for low moisture direct expansion products such as cereals, snacks, nuggets, etc. Also face cutting is used for pasta or tridimensional snacks "pellets".

An external cutter is used for snacks "squares, waves, straight sticks, etc", high moisture extrusion, etc. Other devices can be combination of cutter devices and particle size reducers such as "Fitz mills, roller mills, commitrols, etc. "TVP, TC, etc"

After product is reduced to the proper particle size; it is dried to the proper specifications; typically from 2% - 10% to be shelf stable at normal warehouse conditions; and greater than 12% should be frost or refrigerated.

One additional typical attribute to consider is:

Density: (0.12-0.35 g/cc).

The product is packaged after drying and sifting steps.

**Claims**

1. A soy protein hydrolysate composition, the composition comprising a mixture of polypeptides enriched in a 47 kDa fragment, wherein between about 10% and about 66% of the polypeptides by weight are the 47 kDa fragment.

2. The composition of claim 1, wherein the composition comprises at least one oligopeptide selected from the group consisting of SEQ ID NO:2, SEQ ID NO:3, SEQ ID NO:4, SEQ ID NO:5, SEQ ID NO:6, SEQ ID NO:7, and SEQ ID NO:8.

3. A protein fraction comprising an enriched hydrophobic ß-conglycinin core region.

4. A food product comprising the soy protein hydrolysate composition of claim 1.

5. The food product of claim 4 selected from the group consisting of cheese, emulsified meats, whipped food products, food bars, surimi products, extruded food products, beverages, whole muscle food products, yogurt, puddings and combinations thereof.

6. A process for making a protein fraction comprising:

a. preparing a soy protein slurry using soy flour or soy concentrate derived from the isoelectric precipitation at pH 4.5, and

b. hydrolyzing the soy protein slurry with an enzyme that cleaves proteins at the C-terminus of glutamic acid and aspartic acid to obtain a protein fraction.

7. A process to produce a hydrophobic ß-conglycinin core region enriched protein product wherein the hydrophilic extension region of the α' and α subunits of ß-conglycinin are selectively hydrolyzed from ß-conglycinin by hydrolyzing the ß-conglycinin with an enzyme that cleaves proteins at the C-terminus of glutamic acid and aspartic acid to yield a hydrophobic ß-conglycinin core region enriched protein product.

8. The process of claim 7 wherein the enzyme is a protease.

9. The process of claim 8 wherein the enzyme is a serine protease.

10. The process of claim 9 wherein the enzyme is serine protease from *Bacillus lichenformis.*

11. A process for preparing a protein hydrolysate composition, the process comprising:

a. contacting a protein material with an endopeptidase that cleaves the protein material into mixture of polypeptides enriched in a 47 kDa fragment, the mixture of polypeptides comprising the protein hydrolysate composition, wherein between about 10% and about 66% of the polypeptides by weight are the 47 kDa fragment.

12. The process of claim 11, wherein the endopeptidase is a serine protease.

13. The process of claim 12, wherein the serine protease is from *Bacillus lichenformis.*

14. A food product, the food product comprising a protein hydrolysate composition including a mixtures of polypeptides enriched in a 47 kDa fragment, wherein between about 10% and about 66% of the polypeptides by weight are the 47 kDa fragment.

15. The food product of claim 14, wherein the food product is selected from the group consisting of cheese, surimi products, emulsified meats, whipped fond products, food bars, extruded food products, beverages, whole muscle food products, yogurt, puddings and combinations thereof.

## Patentansprüche

1. Sojaproteinhydrolysatzusammensetzung, wobei die Zusammensetzung eine Mischung von Polypeptiden umfasst, die mit einem 47 kDa-Fragment angereichert ist, wobei etwa 10 % bis etwa 66 % der Polypeptide, auf das Gewicht bezogen, aus dem 47 kDa-Fragment bestehen.

2. Zusammensetzung nach Anspruch 1, wobei die Zusammensetzung mindestens ein Oligopeptid umfasst ausgewählt aus der Gruppe bestehend aus SEQ ID NO:2, SEQ ID NO:3, SEQ ID NO:4, SEQ ID NO:5, SEQ ID NO:6, SEQ ID NO:7 und SEQ ID NO:8.

3. Proteinfraktion umfassend eine angereicherte hydrophobe ß-Conglycinin-Kemregion.

4. Nahrungsmittelprodukt umfassend die Sojaproteinhydrolysatzusammensetzung nach Anspruch 1.

5. Nahrungsmittelprodukt nach Anspruch 4, ausgewählt aus der Gruppe bestehend aus Käse, emulgierten Fleischsorten, aufgeschlagenen Nahrungsmittelprodukten, Nahrungsmittelriegeln, Surimi-Produkten, extrudierten Nahrungsmittelprodukten, Getränken, Ganzmuskelnahrungsmittelprodukten, Joghurt, Süßspeisen und Kombinationen davon.

6. Verfahren für die Herstellung einer Proteinfraktion, umfassend:

a. das Herstellen eines dünnen Sojaproteinbreis unter Anwendung von Sojamehl oder Sojakonzentrat, das aus der isoelektrischen Ausfällung bei einem pH-Wert von 4,5 stammt, und

b. das Hydrolysieren des dünnen Sojaproteinbreis mit einem Enzym, das Proteine am C-Terminus von Glutaminsäure und Asparaginsäure spaltet, um eine Proteinfraktion zu erhalten.

7. Verfahren zum Erzeugen eines mit hydrophober ß-Conglycinin-Kemregion angereicherten Proteinprodukts, wobei die hydrophile Erweiterungsregion der $\alpha$'- und $\alpha$-Untereinheiten von ß-Conglycinin selektiv aus ß-Conglycinin durch Hydrolysieren des ß-Conglycinins mit einem Enzym hydrolysiert wird, das Proteine am C-Terminus von Glutaminsäure und Asparaginsäure spaltet, um ein mit hydrophober ß-Conglycinin-Kemregion angereichertes Proteinprodukt zu ergeben.

8. Verfahren nach Anspruch 7, wobei das Enzym eine Protease ist.

9. Verfahren nach Anspruch 8, wobei das Enzym eine Serinprotease ist.

10. Verfahren nach Anspruch 9, wobei das Enzym Serinprotease aus *Bacillus lichenformis* ist.

11. Verfahren für die Herstellung einer Proteinhydrolysatzusammensetzung, wobei das Verfahren Folgendes umfasst:

a. das Kontaktieren eines Proteinmaterials mit einer Endopeptidase, die das Proteinmaterial in eine Mischung von Polypeptiden spaltet, die mit einem 47 kDa-Fragment angereichert ist, wobei die Mischung von Polypeptiden die Proteinhydrolysatzusammensetzung umfasst, wobei etwa 10 % bis etwa 66 Gew.-% der Polypeptide aus dem 47 kDa-Fragment bestehen.

12. Verfahren nach Anspruch 11, wobei die Endopeptidase eine Serinprotease ist.

13. Verfahren nach Anspruch 12, wobei die Serinprotease aus *Bacillus lichenformis* stammt.

14. Nahrungsmittelprodukt, wobei das Nahrungsmittelprodukt eine Proteinhydrolysatzusammensetzung umfasst, die eine Mischung von Polypeptiden umfasst, die mit einem 47 kDa-Fragment angereichert ist, wobei etwa 10 % bis etwa 66 % der Polypeptide, auf das Gewicht bezogen, aus dem 47 kDa-Fragment bestehen.

15. Nahrungsmittelprodukt nach Anspruch 14, wobei das Nahrungsmittelprodukt aus der Gruppe ausgewählt ist bestehend aus Käse, Surimi-Produkten, emulgierten Fleischsorten, aufgeschlagenen Nahrungsmittelprodukten, Nahrungsmittelriegeln, extrudierten Nahrungsmittelprodukten, Getränken, Ganzmuskelnahrungsmittelprodukten, Joghurt, Süßspeisen und Kombinationen davon.

**Revendications**

1. Composition à base d'hydrolysat de protéines de soja, la composition comprenant un mélange de polypeptides enrichi en un fragment de 47 kDa, dans laquelle entre environ 10 % et environ 66 % des polypeptides, en poids, sont représentés par le fragment de 47 kDa.

2. Composition selon la revendication 1, dans laquelle la composition comprend au moins un oligopeptide choisi dans le groupe constitué par SEQ ID NO:2, SEQ ID NO:3, SEQ ID NO:4, SEQ ID NO:5, SEQ ID NO:6, SEQ ID NO:7 et SEQ ID NO:8.

3. Fraction protéique comprenant une région centrale $\beta$-conglycinine hydrophobe enrichie.

4. Produit alimentaire comprenant la composition à base d'hydrolysat de protéines de soja selon la revendication 1.

5. Produit alimentaire selon la revendication 4, choisi dans le groupe constitué par le fromage, les émulsions de viande, les produits alimentaires fouettés, les barres alimentaires, les produits de type surimi, les produits alimentaires extrudés, les boissons, les produits alimentaires à base de muscle entier, les yaourts, les desserts et des combinaisons de ceux-ci.

6. Procédé de réalisation d'une fraction protéique comprenant :

a. la préparation d'une suspension épaisse de protéines de soja au moyen de farine de soja ou de concentré de soja résultant de la précipitation isoélectrique à un pH de 4,5, et

b. l'hydrolysation de la suspension épaisse de protéines de soja avec une enzyme qui clive les protéines à la terminaison C de l'acide glutamique et de l'acide aspartique pour produire une fraction protéique.

7. Procédé de production d'un produit protéique enrichi en une région centrale β-conglycinine hydrophobe, dans lequel la région d'extension hydrophile des sous-unités α' et α de la β-conglycinine est sélectivement hydrolysée à partir de β-conglycinine en hydrolysant la β-conglycinine avec une enzyme qui clive les protéines à la terminaison C de l'acide glutamique et de l'acide aspartique pour produire un produit protéique enrichi en une région centrale β-conglycinine hydrophobe.

8. Procédé selon la revendication 7, dans lequel l'enzyme est une protéase.

9. Procédé selon la revendication 8, dans lequel l'enzyme est une sérine protéase.

10. Procédé selon la revendication 9, dans lequel l'enzyme est la sérine protéase de *Bacillus lichenformis.*

11. Procédé de préparation d'une composition à base d'hydrolysat de protéines, le procédé comprenant :

a. la mise en contact d'une matière protéique avec une endopeptidase clivant la matière protéique en un mélange de polypeptides enrichi en un fragment de 47 kDa, le mélange de polypeptides comprenant la composition à base d'hydrolysat de protéines, dans lequel entre environ 10 % et environ 66 % des polypeptides, en poids, sont représentés par le fragment de 47 kDa.

12. Procédé selon la revendication 11, dans lequel l'endopeptidase est une sérine protéase.

13. Procédé selon la revendication 12, dans lequel la sérine protéase est issue de *Bacillus lichenformis.*

14. Produit alimentaire, le produit alimentaire comprenant une composition à base d'hydrolysat de protéines comportant un mélange de polypeptides enrichi en un fragment de 47 kDa, dans lequel entre environ 10 % et environ 66 % des polypeptides, en poids, sont représentés par le fragment de 47 kDa.

15. Produit alimentaire selon la revendication 14, dans lequel le produit alimentaire est choisi dans le groupe constitué par le fromage, les produits de type surimi, les émulsions de viande, les produits alimentaires fouettés, les barres alimentaires, les produits alimentaires extrudés, les boissons, les produits alimentaires à base de muscle entier, les yaourts, les desserts et des combinaisons de ceux-ci.

**Figure 1**

## Figure 2

# Figure 3

Figure 4

## Figure 5

## Figure 6

**Figure 7**

## Figure 8

## Figure 9

```
1      vekeeceege   iprprprpqh   perepqqpge   keededeqpr   pipfprpqpr

51     qeeeheqree   qewprkeekr   gekgseeede   dedeeqderq   fpfprpphqk

101    eerkqeeded   eeqqresees   edselrrhkn   knpflfgsnr   fetlfkngyg

151    rirvlqrfnq   rspqlqnlrd   yrilefnskp   ntlllpnhad   adylivilng

201    tailslvnnd   drdsyrlqsg   dalrvpsgtt   yyvvnpdnne   nlrlitlaip

251    vnkpgrfesf   flssteaggs   ylggfsrnil   easydtkfee   inkvlfsree

301    gggggegrlg   esviveiske   giralskrak   sssrktisse   dkpfnlrsrd

351    piysnklgkf   feitpeknpq   lrdldiflsi   vdmnegalll   phfnskaivi

401    lvinegdani   elvglkeggq   egggeegple   vrkyraelse   qdifvipagy

451    pvvvnatsnl   nffaiginae   nnqrnflags   qdnvisqips   qvgelafpgs

501    aqavekllkn   qresyfvdaq   pkkkeegnkg   rkgplssilr   afy
```

**Figure 10**

## Figure 11

## Figure 12

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61141941 B
- WO 9113554 A1
- US 4266031 A
- US 5874278 A
- US 5459064 A
- WO 0116285 A
- WO 9213964 A
- WO 9113553 A
- WO 9113554 A
- WO 9628542 A

**Non-patent literature cited in the description**

- **KARLIN ; ALTSCHUL.** *Proc. Natl. Acad. Sci. USA,* 1990, vol. 87, 2264-2268
- **JENS ADLER-NISSEN.** Determination of the Degree of Hydrolysis of Food Protein Hydrolysates by Trinitrobenzenesulfonic Acid. *J. Agric Food Chem.,* 1979, vol. 1 27 (6), 1256-1262